(19) **Europäisches Patentamt / European Patent Office / Office européen des brevets**

(11) **EP 4 435 492 A1**

(12) **EUROPEAN PATENT APPLICATION**
published in accordance with Art. 153(4) EPC

(43) Date of publication:
**25.09.2024 Bulletin 2024/39**

(21) Application number: **23795158.7**

(22) Date of filing: **19.04.2023**

(51) International Patent Classification (IPC):
**G02B 27/01** (2006.01)      **A61B 3/11** (2006.01)

(52) Cooperative Patent Classification (CPC):
**A61B 3/11; G02B 27/00; G02B 27/01**

(86) International application number:
**PCT/CN2023/089307**

(87) International publication number:
**WO 2023/207725 (02.11.2023 Gazette 2023/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.04.2022 CN 202210451373**

(71) Applicant: **Huawei Technologies Co., Ltd.**
**Shenzhen, Guangdong 518129 (CN)**

(72) Inventors:
- XU, Yue
  **Shenzhen, Guangdong 518129 (CN)**
- JIN, Yunfeng
  **Shenzhen, Guangdong 518129 (CN)**
- ZHU, Shuaishuai
  **Shenzhen, Guangdong 518129 (CN)**

(74) Representative: **Maiwald GmbH**
**Engineering**
**Elisenhof**
**Elisenstrasse 3**
**80335 München (DE)**

(54) **INTER-PUPILARY DISTANCE ADJUSTMENT METHOD, HEAD-MOUNTED DISPLAY DEVICE AND READABLE STORAGE MEDIUM**

(57) An inter pupillary distance adjustment method, a head-mounted display device, and a readable storage medium are provided. The head-mounted display device includes an IPD adjustment mechanism (36) and two optical display modules (33), and the IPD adjustment mechanism (36) is configured to drive the two optical display modules (33) to move. The inter pupillary distance adjustment method includes: after a user wears the head-mounted display device, controlling the IPD adjustment mechanism (36) to move by a first distance in a first direction, where the first distance is used to eliminate a backlash error of the IPD adjustment mechanism (36); and controlling the IPD adjustment mechanism (36) to operate and move by a second distance in a second direction, where the IPD adjustment mechanism (36) drives the two optical display modules (33) to move, and a distance between the two optical display modules (33) after the movement matches an inter pupillary distance of the user. The IPD adjustment mechanism (36) is controlled to operate, to drive the two optical display modules (33) to move. This considers the backlash error of a mechanical structure in the IPD adjustment mechanism (36), and implements automatic adjustment on an inter pupillary distance of the head-mounted display device, and improves convenience and accuracy of inter pupillary distance adjustment.

FIG. 13A

## Description

This application claims priority to Chinese Patent Application No. 202210451373.5, filed with the China National Intellectual Property Administration on April 26, 2022 and entitled "INTER PUPILLARY DISTANCE ADJUSTMENT METHOD, HEAD-MOUNTED DISPLAY DEVICE, AND READABLE STORAGE MEDIUM", which is incorporated herein by reference in its entirety.

## TECHNICAL FIELD

Embodiments of this application relate to the field of terminal technologies, and in particular, to an inter pupillary distance adjustment method, a head-mounted display device, and a readable storage medium.

## BACKGROUND

A head-mounted display device is a display device worn on a head of a user, and may provide a new visual environment for the user. With development of display technologies, for example, an augmented reality (augmented reality, AR) technology, a virtual reality (virtual reality, VR) technology, or a mixed reality (mixed reality, MR) technology, the head-mounted display device may provide the user with a feeling of closing to a real scene and is popular with users.

When the user wears the head-mounted display device, because inter pupillary distances (inter pupillary distance, IPD) of different people are usually different, a distance between two lens tubes of the head-mounted display device needs to be adjusted.

Currently, IPD adjustment is implemented manually and mechanically, and the user manually operates an adjustment mechanism on the head-mounted display device, to adjust the distance between the two lens tubes. An operation of the user is complex, and an adjustment effect is poor.

## SUMMARY

Embodiments of this application provide an inter pupillary distance adjustment method, a head-mounted display device, and a readable storage medium, to improve convenience and accuracy of performing inter pupillary distance adjustment by the head-mounted display device.

According to a first aspect, an inter pupillary distance adjustment method is provided, applied to a head-mounted display device. The head-mounted display device includes an IPD adjustment mechanism and two optical display modules, and the IPD adjustment mechanism is configured to drive the two optical display modules to move. The inter pupillary distance adjustment method includes: after a user wears the head-mounted display device, controlling the IPD adjustment mechanism to move by a first distance in a first direction, where the first distance is used to eliminate a backlash error of the IPD adjustment mechanism; and controlling the IPD adjustment mechanism to operate and move by a second distance in a second direction, where the IPD adjustment mechanism drives the two optical display modules to move, and a distance between the two optical display modules after the movement matches an inter pupillary distance of the user. In some embodiments, the two optical display modules are driven to move until a determining module determines that a deviation between an inter pupillary distance of the head-mounted display device and the inter pupillary distance of the user falls within a first error range. For example, the first error range is less than or equal to A, and a value of A is less than or equal to 1 millimeter.

According to the inter pupillary distance adjustment method provided in the first aspect, the IPD adjustment mechanism is controlled to move by the first distance in the first direction, so that the backlash error that may be introduced by a mechanical structure in the IPD adjustment mechanism may be eliminated in a process of adjusting the inter pupillary distance by the head-mounted display device, and accuracy of inter pupillary distance adjustment is improved. In addition, the IPD adjustment mechanism is continuously controlled to move by the second distance in the second direction, and the two optical display modules can be driven to move. In this way, the inter pupillary distance of the head-mounted display device can be automatically adjusted, manual and mechanical adjustment of the user is avoided, and convenience and accuracy of adjusting the inter pupillary distance by the head-mounted display device can be improved. The second direction is also a target movement direction in this embodiment of this application, and may be a direction in which the two optical display modules move close to or away from each other. The first distance is greater than or equal to 0. The first direction is a direction in which the two optical display modules move close to or away from each other.

In a possible implementation, the first direction is opposite to the second direction, and the first direction is a direction in which the two optical display modules move close to or away from each other.

According to the inter pupillary distance adjustment method provided in this implementation, the IPD adjustment mechanism is first controlled to move by the first distance in the first direction, the first direction is a direction opposite to the second direction or a direction opposite to the target movement direction in this embodiment of this application, the first distance is greater than 0, and the first distance is used to eliminate influence of the backlash error introduced by the mechanical structure in the IPD adjustment mechanism on inter pupillary distance adjustment of the device. Then, the IPD adjustment mechanism is controlled to operate in an opposite direction, that is, to move by the second distance in the second direction (the target movement direction), to drive the two optical display modules to move in the second direction, thereby improving precision and accuracy of inter pupillary distance adjustment of the device. This method is applicable to a scenario in which an accumulated error may be introduced during a plurality of times of forward/reverse adjustment.

In a possible implementation, the first direction is a preset direction, and the preset direction is the direction in which the two optical display modules move close to or away from each other.

In the inter pupillary distance adjustment method provided in this implementation, both the first direction and the second direction are preset directions, and the first direction is opposite to the second direction. For example, the first direction is a direction in which the two optical display modules move close to each other, and the second direction is a direction in which the two optical display modules move away from each other. Alternatively, the first direction is a direction in which the two optical display modules move away from each other, and the second direction is a direction in which the two optical display modules move close to each other. The implementation is simple.

In a possible implementation, the controlling the IPD adjustment mechanism to move by a first distance in a first direction includes: controlling the IPD adjustment mechanism to operate and move by the first distance in the first direction, to drive the two optical display modules to move beyond a preset distance or to move to an inter pupillary distance limit location.

In this implementation, the IPD adjustment mechanism is controlled to move by the first distance in the first direction, that is, the two optical display modules are driven to move by the first distance in the first direction. In a scenario, the two optical display modules do not reach the inter pupillary distance limit location after moving by the first distance, and the first distance exceeds the preset distance. Moving by the preset distance can eliminate the backlash error of the IPD adjustment mechanism, thereby improving accuracy of inter pupillary distance adjustment. In another scenario, the two optical display modules first move to the inter pupillary distance limit location, and then move in one direction from the inter pupillary distance limit location, thereby eliminating the backlash error of the IPD adjustment mechanism and improving accuracy of inter pupillary distance adjustment.

In a possible implementation, the first direction is the same as the second direction, and the first direction is a direction in which the two optical display modules move close to or away from each other.

In the inter pupillary distance adjustment method provided in this implementation, the first direction is the same as the second direction, both the first direction and the second direction are target movement directions in this embodiment of this application. In addition, there is one movement direction in an inter pupillary distance adjustment process. This is simple to implement. The IPD adjustment mechanism is controlled to move by the first distance in the first direction, so that the backlash error of the IPD adjustment mechanism is eliminated, and the accuracy of inter pupillary distance adjustment is improved.

In a possible implementation, the first direction and the second direction are opposite to an operation direction of the IPD adjustment mechanism during a previous inter pupillary distance adjustment of the head-mounted display device, and the first distance is 0; and the controlling the IPD adjustment mechanism to move by a first distance in a first direction includes: controlling the IPD adjustment mechanism to operate for first duration in the first direction, where the first duration is used to eliminate the backlash error of the IPD adjustment mechanism.

In the inter pupillary distance adjustment method provided in this implementation, a comparison needs to be made to determine whether a first direction (or a second direction, or a target movement direction) in a current inter pupillary distance adjustment is the same as an inter pupillary distance adjustment direction in a previous inter pupillary distance adjustment. If adjustment directions in two consecutive inter pupillary distance adjustments are opposite, influence of the backlash error caused by the mechanical structure in the IPD adjustment mechanism on inter pupillary distance adjustment during forward/reverse adjustment needs to be considered. During the current inter pupillary distance adjustment, the IPD adjustment mechanism is first controlled to operate for the first duration in the first direction, to eliminate the backlash error introduced by the mechanical structure in the IPD adjustment mechanism, but a distance of the IPD adjustment mechanism or a distance between the two optical display modules does not change, that is, the first distance is 0. Then, the IPD adjustment mechanism may be continuously controlled to operate and move by the second distance in the first direction, to implement automatic inter pupillary distance adjustment of the head-mounted display device.

In a possible implementation, the IPD adjustment mechanism includes a motor, a gear connected to the motor, and a rack connected to the gear, and the rack is further connected to the optical display module; and the controlling the IPD adjustment mechanism to operate for first duration in the first direction includes: controlling the motor to operate for the first duration in the first direction, to drive the gear to rotate for the first duration.

In this implementation, the mechanical structure in the IPD adjustment mechanism includes the gear and the rack. Due to a gap between teeth of the gear and the rack, a backlash error occurs. Because an inter pupillary distance adjustment direction (a first direction, or a second direction, or a target movement direction) in a current inter pupillary distance adjustment is opposite to an inter pupillary distance adjustment direction in a previous inter pupillary distance adjustment, when the motor is first controlled to operate for the first duration in the first direction, the backlash error between the gear and the rack is eliminated, but a distance of the IPD adjustment mechanism or a distance between the two optical display modules does not change, that is, the first distance is 0. Then, the IPD adjustment mechanism may be continuously controlled to operate and move by the second distance in the first direction, to implement automatic inter pupillary distance adjustment of the head-mounted display device.

In a possible implementation, the first direction and the second direction are the same as an operation direction of the IPD adjustment mechanism during a previous inter pupillary distance adjustment of the head-mounted display device, and the first distance is 0; and the controlling the IPD adjustment mechanism to move by a first distance in a first direction includes: controlling the IPD adjustment mechanism not to operate.

In the inter pupillary distance adjustment method provided in this implementation, a comparison needs to be made to determine whether a first direction (or a second direction, or a target movement direction) in a current inter pupillary distance adjustment is the same as an inter pupillary distance adjustment direction in a previous inter pupillary distance adjustment. If adjustment directions in two consecutive inter pupillary distance adjustments are the same, influence of the backlash error caused by the mechanical structure in the IPD adjustment mechanism on inter pupillary distance adjustment during forward/reverse adjustment does not need to be considered. During a current inter pupillary distance adjustment, the IPD adjustment mechanism can be directly controlled to operate in the first direction. It may be understood that the IPD adjustment mechanism is controlled not to operate, and the first distance is 0. Then, the IPD adjustment mechanism may be continuously controlled to operate and move by the second distance in the first direction, to implement automatic inter pupillary distance adjustment of the head-mounted display device.

In a possible implementation, before the controlling the IPD adjustment mechanism to move by a first distance in a first direction, the inter pupillary distance adjustment method further includes: obtaining inter pupillary distance adjustment information, where the inter pupillary distance adjustment information represents an inter pupillary distance adjustment value adjusted by the head-mounted display device; and determining the first direction and/or the second direction based on the inter pupillary distance adjustment information.

In a possible implementation, the head-mounted display device further includes an eye tracking apparatus, and the obtaining inter pupillary distance adjustment information includes: obtaining an inter pupillary distance offset of a single eye by using the eye tracking apparatus, where the inter pupillary distance offset of the single eye indicates a deviation between a pupil of the single eye and a corresponding optical display module.

According to the inter pupillary distance adjustment method provided in this implementation, the inter pupillary distance adjustment information includes the inter pupillary distance offset of the single eye, and an inter pupillary distance adjustment value that needs to be adjusted by the head-mounted display device may be automatically obtained by using the eye tracking apparatus, thereby improving accuracy of obtaining the inter pupillary distance adjustment information. In addition, compared with adjusting an inter pupillary distance based on a viewing feeling of a human eye of a user, this manner improves accuracy of performing inter pupillary distance adjustment by the head-mounted display device.

In a possible implementation, the obtaining an inter pupillary distance offset of a single eye by using the eye tracking apparatus includes: performing eye tracking on a left eye or a right eye of a person by using the eye tracking apparatus, and determining an obtained inter pupillary distance offset of the left eye or an obtained inter pupillary distance offset of the right eye as the inter pupillary distance offset of the single eye.

In this implementation, there is only one inter pupillary distance offset of the single eye, which is specifically the inter pupillary distance offset of the left eye or the inter pupillary distance offset of the right eye. Because structures of human eyes and the head-mounted display device are usually symmetrical, performing eye tracking on a single eye to obtain an inter pupillary distance offset of the single eye is simple to implement.

In a possible implementation, the obtaining an inter pupillary distance offset of a single eye by using the eye tracking apparatus includes: separately performing eye tracking on a left eye and a right eye of a person by using the eye tracking apparatus, and determining the inter pupillary distance offset of the single eye based on an obtained inter pupillary distance offset of the left eye and an obtained inter pupillary distance offset of the right eye.

In this implementation, eye tracking is separately performed on the left eye and the right eye of the person by using the eye tracking apparatus, and the inter pupillary distance offset of the single eye is determined based on the obtained inter pupillary distance offset of the left eye and the obtained inter pupillary distance offset of the right eye, thereby improving accuracy of determining the inter pupillary distance offset.

In a possible implementation, the determining the inter pupillary distance offset of the single eye based on an obtained inter pupillary distance offset of the left eye and an obtained inter pupillary distance offset of the right eye includes: determining the inter pupillary distance offset of the left eye and the inter pupillary distance offset of the right eye as the inter pupillary distance offset of the single eye; or determining an average value of the inter pupillary distance offset of the left eye and the inter pupillary distance offset of the right eye as the inter pupillary distance offset of the single eye.

In this implementation, a scenario is that there are two inter pupillary distance offsets of the single eye that include the inter pupillary distance offset of the left eye and the inter pupillary distance offset of the right eye.

Subsequently, in a process of adjusting the inter pupillary distance of the head-mounted display device, locations of optical display modules corresponding to the left eye and the right eye may be separately moved. This improves accuracy and flexibility of inter pupillary distance adjustment of the device. In the other scenario, there is only one inter pupillary distance offset of the single eye. An average value is calculated. This improves accuracy of determining the inter pupillary distance offset of the single eye.

In a possible implementation, the head-mounted display device further includes an IPD adjustment sensor, and a detection value of the IPD adjustment sensor represents an inter pupillary distance of the head-mounted display device; and the obtaining inter pupillary distance adjustment information further includes: obtaining a current detection value of the IPD adjustment sensor; and determining a target inter pupillary distance value and/or a target detection value based on a calibration table, the inter pupillary distance offset of the single eye, and the current detection value, where the calibration table indicates a correspondence between the detection value of the IPD adjustment sensor and the inter pupillary distance of the head-mounted display device.

According to the inter pupillary distance adjustment method provided in this implementation, the inter pupillary distance adjustment information includes the target inter pupillary distance value and/or the target detection value, and an inter pupillary distance adjustment value may be obtained by using the IPD adjustment sensor and the calibration table. Subsequently, it may be determined, based on this, whether inter pupillary distance adjustment can be ended, thereby improving accuracy of inter pupillary distance adjustment.

In a possible implementation, the obtaining inter pupillary distance adjustment information includes: obtaining a target inter pupillary distance value entered by the user.

In a possible implementation, the obtaining a target inter pupillary distance value entered by the user includes: obtaining the target inter pupillary distance value entered by the user through voice.

In a possible implementation, the obtaining a target inter pupillary distance value entered by the user includes: displaying a first interface; and obtaining the target inter pupillary distance value entered by the user in the first interface.

In a possible implementation, the head-mounted display device further includes the eye tracking apparatus, and that the deviation between the inter pupillary distance of the head-mounted display device and the inter pupillary distance of the user falls within the first error range includes: $|\delta x| \leq k$, where $\delta x$ represents an inter pupillary distance offset that is of the single eye at a current moment and that is obtained by using the eye tracking apparatus, and k represents a preset inter pupillary distance offset error.

According to the inter pupillary distance adjustment method provided in this implementation, determining whether inter pupillary distance adjustment can be ended is implemented by using the eye tracking apparatus. If the inter pupillary distance offset of the single eye falls within the first error range, it can be determined that inter pupillary distance adjustment of the device is completed.

In a possible implementation, the head-mounted display device further includes the IPD adjustment sensor, and the detection value of the IPD adjustment sensor represents the inter pupillary distance of the head-mounted display device; and that the deviation between the inter pupillary distance of the head-mounted display device and the inter pupillary distance of the user falls within the first error range includes: $|V2-V1| \leq \delta V$, and/or $|L2-L1| \leq \delta L$, where V1 represents a first detection value of the IPD adjustment sensor at a current moment, V2 represents the target detection value corresponding to the target inter pupillary distance of the head-mounted display device, L1 represents a first inter pupillary distance value that corresponds to the first detection value V1 and that is determined according to the calibration table, L2 represents the target inter pupillary distance value, $\delta V$ represents a preset detection value error, $\delta L$ represents a preset inter pupillary distance error, and the calibration table indicates the correspondence between the detection value of the IPD adjustment sensor and the inter pupillary distance of the head-mounted display device.

According to the inter pupillary distance adjustment method provided in this implementation, whether inter pupillary distance adjustment can be ended is determined by using the IPD adjustment sensor.

In a possible implementation, the calibration table includes a first calibration table and a second calibration table, the first calibration table corresponds to a movement direction in which the two optical display modules move close to each other, and the second calibration table corresponds to a movement direction in which the two optical display modules move away from each other.

In a possible implementation, the inter pupillary distance adjustment method further includes: outputting prompt information, where the prompt information indicates that inter pupillary distance adjustment of the head-mounted display device ends.

According to the inter pupillary distance adjustment method provided in this implementation, after automatic inter pupillary distance adjustment is completed, the prompt information may be output, to inform the user that inter pupillary distance adjustment ends, thereby improving user experience.

In a possible implementation, the prompt information further includes an inter pupillary distance value of the head-mounted display device after the inter pupillary distance adjustment.

According to the inter pupillary distance adjustment method provided in this implementation, an inter pupillary distance value obtained after automatically adjustment is output to the user, so that the user can learn of the inter pupillary distance of the user, thereby improving user experience.

According to a second aspect, an inter pupillary distance adjustment apparatus is provided, and may include: a processing module, configured to: after a user wears a head-mounted display device, control an IPD adjustment mechanism to move by a first distance in a first direction, where the first distance is used to eliminate a backlash error of the IPD adjustment mechanism; and control the IPD adjustment mechanism to operate and move by a second distance in a second direction, where the IPD adjustment mechanism drives two optical display modules to move, and a distance between the two optical display modules after the movement matches an inter pupillary distance of the user. In some embodiments, the two optical display modules are driven to move until a determining module determines that a deviation between an inter pupillary distance of the head-mounted display device and the inter pupillary distance of the user falls within a first error range. For example, the first error range is less than or equal to A, and a value of A is less than or equal to 1 millimeter.

In a possible implementation, the first direction is opposite to the second direction, and the first direction is a direction in which the two optical display modules move close to or away from each other.

In a possible implementation, the first direction is a preset direction, and the preset direction is the direction in which the two optical display modules move close to or away from each other.

In a possible implementation, the processing module is configured to: control the IPD adjustment mechanism to operate and move by the first distance in the first direction, to drive the two optical display modules to move beyond a preset distance or to move to an inter pupillary distance limit location.

In a possible implementation, the first direction is the same as the second direction, and the first direction is a direction in which the two optical display modules move close to or away from each other.

In a possible implementation, the first direction and the second direction are opposite to an operation direction of the IPD adjustment mechanism during a previous inter pupillary distance adjustment of the head-mounted display device, and the first distance is 0; and the processing unit is configured to control the IPD adjustment mechanism to operate for first duration in the first direction, where the first duration is used to eliminate the backlash error of the IPD adjustment mechanism.

In a possible implementation, the IPD adjustment mechanism includes a motor, a gear connected to the motor, and a rack connected to the gear, and the rack is further connected to the optical display module; and the processing module is configured to: control the motor to operate for the first duration in the first direction, to drive the gear to rotate for the first duration.

In a possible implementation, the first direction and the second direction are the same as an operation direction of the IPD adjustment mechanism during a previous inter pupillary distance adjustment of the head-mounted display device, and the first distance is 0; and the processing module is configured to: control the IPD adjustment mechanism not to operate.

In a possible implementation, the processing unit is further configured to: before controlling the IPD adjustment mechanism to move by the first distance in the first direction, obtain inter pupillary distance adjustment information, where the inter pupillary distance adjustment information represents an inter pupillary distance adjustment value adjusted by the head-mounted display device; and determine the first direction and/or the second direction based on the inter pupillary distance adjustment information.

In a possible implementation, the head-mounted display device further includes an eye tracking apparatus, and the processing unit is configured to obtain an inter pupillary distance offset of a single eye by using the eye tracking apparatus, where the inter pupillary distance offset of the single eye indicates a deviation between a pupil of the single eye and a corresponding optical display module.

In a possible implementation, the processing unit is configured to: perform eye tracking on a left eye or a right eye of a person by using the eye tracking apparatus, and determine an obtained inter pupillary distance offset of the left eye or an obtained inter pupillary distance offset of the right eye as the inter pupillary distance offset of the single eye.

In a possible implementation, the processing unit is configured to: separately perform eye tracking on a left eye and a right eye of a person by using the eye tracking apparatus, and determine the inter pupillary distance offset of the single eye based on an obtained inter pupillary distance offset of the left eye and an obtained inter pupillary distance offset of the right eye.

In a possible implementation, the processing unit is configured to: determine the inter pupillary distance offset of the left eye and the inter pupillary distance offset of the right eye as the inter pupillary distance offset of the single eye; or determine an average value of the inter pupillary distance offset of the left eye and the inter pupillary distance offset of the right eye as the inter pupillary distance offset of the single eye.

In a possible implementation, the head-mounted display device further includes an IPD adjustment sensor, and a detection value of the IPD adjustment sensor represents an inter pupillary distance of the head-mounted display device; and the processing unit is further configured to: obtain a current detection value of the IPD adjustment sensor; and determine a target inter pupillary distance value and/or a target detection value based on a calibration table, the inter pupillary distance offset of the single eye, and the current detection value, where the calibration table indicates a correspondence between the detection value of the IPD adjustment sensor and the inter pupillary distance of the head-mounted display device.

In a possible implementation, the processing module is configured to obtain a target inter pupillary distance value entered by the user.

In a possible implementation, the processing module is configured to obtain the target inter pupillary distance value entered by the user through voice.

In a possible implementation, the processing module is configured to: display a first interface; and obtain the target inter pupillary distance value entered by the user in the first interface.

In a possible implementation, the head-mounted display device further includes the eye tracking apparatus, and that the deviation between the inter pupillary distance of the head-mounted display device and the inter pupillary distance of the user falls within the first error range includes: $|\delta x| \leq k$, where $\delta x$ represents an inter pupillary distance offset that is of the single eye at a current moment and that is obtained by using the eye tracking apparatus, and k represents a preset inter pupillary distance offset error.

In a possible implementation, the head-mounted display device further includes the IPD adjustment sensor, and the detection value of the IPD adjustment sensor represents the inter pupillary distance of the head-mounted display device; and that the deviation between the inter pupillary distance of the head-mounted display device and the inter pupillary distance of the user falls within the first error range includes: $|V2-V1| \leq \delta V$, and/or $|L2-L1| \leq \delta L$, where V1 represents a first detection value of the IPD adjustment sensor at a current moment, V2 represents the target detection value corresponding to the target inter pupillary distance of the head-mounted display device, L1 represents a first inter pupillary distance value that corresponds to the first detection value V1 and that is determined according to the calibration table, L2 represents the target inter pupillary distance value, $\delta V$ represents a preset detection value error, $\delta L$ represents a preset inter pupillary distance error, and the calibration table indicates the correspondence between the detection value of the IPD adjustment sensor and the inter pupillary distance of the head-mounted display device.

In a possible implementation, the calibration table includes a first calibration table and a second calibration table, the first calibration table corresponds to a movement direction in which the two optical display modules move close to each other, and the second calibration table corresponds to a movement direction in which the two optical display modules move away from each other.

In a possible implementation, the processing unit is further configured to output prompt information, where the prompt information indicates that inter pupillary distance adjustment of the head-mounted display device ends.

In a possible implementation, the prompt information further includes an inter pupillary distance value

of the head-mounted display device after the inter pupillary distance adjustment.

According to a third aspect, a head-mounted display device is provided, including an IPD adjustment mechanism, two optical display modules, a processor, and a memory. The IPD adjustment mechanism is configured to drive the two optical display modules to move. The processor is configured to: be coupled to the memory, read instructions in the memory, and enable, according to the instructions, the head-mounted display device to perform the method provided in the first aspect of this application.

In a possible implementation, the head-mounted display device further includes an eye tracking apparatus, and the eye tracking apparatus includes at least one light source and at least one camera module. The at least one light source is used for emitting light to a human eye, and the at least one camera module is configured to obtain a part of light reflected after the at least one light source irradiates the eye.

In a possible implementation, a light source and a camera module are correspondingly disposed for each optical display module, or a light source and a camera module are correspondingly disposed in one of the two optical display modules.

In a possible implementation, the optical display module includes an optical module and a display component, and the optical module includes at least one optical component; and the camera module is located between the optical module and the human eye, the camera module is located between two adjacent optical components in the optical module, or the camera module is located between the optical module and the display component.

In a possible implementation, the optical module includes a first optical component, and a distance between the first optical component and the display component is adjustable; and the camera module is located between the first optical component and the display component.

In a possible implementation, the eye tracking apparatus further includes a focal length measurement component, and the focal length measurement component is configured to detect focal length information of the optical display module.

In a possible implementation, the focal length measurement component includes a Hall effect sensor, an infrared laser ranging unit, or a slide rheostat.

In a possible implementation, the head-mounted display device further includes an IPD adjustment sensor, and a detection value of the IPD adjustment sensor represents an inter pupillary distance of the head-mounted display device.

In a possible implementation, the IPD adjustment sensor includes a Hall effect sensor or a slide rheostat.

In a possible implementation, the IPD adjustment mechanism includes a motor and an IPD adjustment mechanical structure connected to the motor, the IPD adjustment mechanical structure is connected to the two optical display modules, and the IPD adjustment mechanical structure is configured to drive, under driving of the motor, the two optical display modules to move.

In a possible implementation, there is one motor, and the IPD adjustment mechanical structure is configured to drive, under driving of the motor, the two optical display modules to move simultaneously.

In a possible implementation, there are two motors, each motor corresponds to an IPD adjustment mechanical structure, and the two IPD adjustment mechanical structures are connected to the two optical display modules in a one-to-one correspondence; and the IPD adjustment mechanical structure is configured to drive, under driving of the corresponding motor, the corresponding optical display module to move.

In a possible implementation, the IPD adjustment mechanical structure includes a gear and a rack, the gear is separately connected to the motor and the rack, and the rack is connected to the optical display module.

According to a fourth aspect, an IPD adjustment mechanism is provided, including a motor and an IPD adjustment mechanical structure connected to the motor. The motor is configured to: receive a control signal, and perform displacement based on the control signal. The IPD adjustment mechanical structure is configured to: connect to two optical display modules in the head-mounted display device, and drive, under driving of the motor, the two optical display modules to move. In this embodiment of this application, the control signal may be a control signal sent by a processor of the head-mounted display device, the control signal is used to drive the motor to move, to drive the IPD adjustment mechanical structure to drive the two optical display modules to move, and adjust an inter pupillary distance of the head-mounted display device.

In a possible implementation, there is one motor, and the IPD adjustment mechanical structure is configured to drive, under driving of the motor, the two optical display modules to move simultaneously.

In a possible implementation, there are two motors, each motor corresponds to an IPD adjustment mechanical structure, and the two IPD adjustment mechanical structures are connected to the two optical display modules in a one-to-one correspondence; and the IPD adjustment mechanical structure is configured to drive, under driving of the corresponding motor, the corresponding optical display module to move.

In a possible implementation, the IPD adjustment mechanical structure includes a gear and a rack, the gear is separately connected to the motor and the rack, and the rack is connected to the optical display module.

In a possible implementation, the motor is configured to operate for first duration in a first direction, the first duration is used to eliminate a backlash error between the gear and the rack, and the first direction is a direction in which the two optical display modules move close to or away from each other. A previous movement direction of the motor is opposite to the first direction.

According to a fifth aspect, a program is provided. When the program is executed by a processor, the method provided in the first aspect is performed.

According to a sixth aspect, a computer-readable storage medium is provided. The computer-readable storage medium stores instructions, and when the instructions are run on a computer or a processor, the method provided in the first aspect is implemented.

According to a seventh aspect, a program product is provided. The program product includes a computer program, the computer program is stored in a readable storage medium, at least one processor of a device may read the computer program from the readable storage medium, and the at least one processor executes the computer program, to enable the device to implement the method provided in the first aspect.

**BRIEF DESCRIPTION OF DRAWINGS**

FIG. 1 is a diagram of a structure of VR glasses according to an embodiment of this application;
FIG. 2A and FIG. 2B each are a diagram of a case in which a user inter pupillary distance does not match a device inter pupillary distance according to an embodiment of this application;
FIG. 3 is a diagram of a structure of a head-mounted display device according to an embodiment of this application;
FIG. 4A and FIG. 4B each are a diagram of a structure of an optical display module according to an embodiment of this application;
FIG. 5A and FIG. 5B each are a diagram of a structure of an IPD adjustment mechanism according to an embodiment of this application;
FIG. 6 is a diagram of a structure of an eye tracking apparatus according to an embodiment of this application;
FIG. 7 is a diagram of composition of a human eye according to an embodiment of this application;
FIG. 8 is a diagram of a principle of corneal reflection-based eye tracking according to an embodiment of this application;
FIG. 9 is a diagram of a calibration curve of an IPD distance and a magnetic induction intensity according to an embodiment of this application;
FIG. 10A to FIG. 10D are diagrams of a group of interfaces according to an embodiment of this application;
FIG. 11 is a flowchart of an inter pupillary distance adjustment method according to an embodiment of this application;
FIG. 12 is another flowchart of an inter pupillary distance adjustment method according to an embodiment of this application;

FIG. 13A and FIG. 13B are a group of diagrams of inter pupillary distance adjustment according to an embodiment of this application;

FIG. 14A and FIG. 14B are another group of diagrams of inter pupillary distance adjustment according to an embodiment of this application;

FIG. 15 is still another flowchart of an inter pupillary distance adjustment method according to an embodiment of this application;

FIG. 16 is still another flowchart of an inter pupillary distance adjustment method according to an embodiment of this application;

FIG. 17 is still another flowchart of an inter pupillary distance adjustment method according to an embodiment of this application;

FIG. 18 is still another flowchart of an inter pupillary distance adjustment method according to an embodiment of this application;

FIG. 19 is still another flowchart of an inter pupillary distance adjustment method according to an embodiment of this application;

FIG. 20 is still another flowchart of an inter pupillary distance adjustment method according to an embodiment of this application; and

FIG. 21 is a diagram of a structure of an inter pupillary distance adjustment apparatus according to an embodiment of this application.

## DESCRIPTION OF EMBODIMENTS

The following describes embodiments of this application with reference to accompanying drawings.

An inter pupillary distance adjustment method provided in embodiments of this application is applicable to a head-mounted display device. A type, a name, and a structure of the head-mounted display device are not limited in embodiments of this application. For example, the head-mounted display device may be glasses using an AR technology, a VR technology, or an MR technology, and is configured to provide a virtual display function for a user.

For ease of description, an example in which the head-mounted display device is VR glasses is used in embodiments of this application.

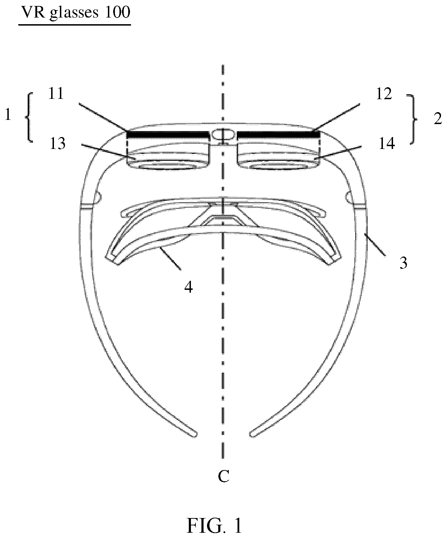

For example, FIG. 1 is a diagram of a structure of VR glasses according to an embodiment of this application. As shown in FIG. 1, the VR glasses 100 include an optical display module 1 and an optical display module 2. Optionally, the VR glasses 100 further include a temple 3 and a supporting part 4. The temple 3 is configured to support the VR glasses 100, so that the VR glasses 100 can sit over ears of a user, and the supporting part 4 is configured to support the VR glasses 100, so that the VR glasses 100 can sit over a nose of the user, to ensure that the user can stably wear the VR glasses. The optical display module 1 includes a display component 11 and an optical module 13. The optical display module 2 includes a display component 12 and an optical module 14. The optical module 13 and the optical module 14 are symmetrical relative to a center line of a human face or a center line C of the VR glasses 100. Optionally, the center line of the human face may be a perpendicular bisector between a left eye and a right eye of a person. The center line of the VR glasses 100 may be a center line of the temple 3 or the supporting part 4.

When the user wears the VR glasses 100, the optical display module 1 is configured to display an image to a left eye of the user, and the optical display module 2 is configured to display an image to a right eye of the user. For the optical display module 1, the optical module 13 is close to the left eye of the person, and the display component 11 is far away from the left eye of the person. The display component 11 has a display function. When the display component 11 displays an image, light emitted by the display component 11 is converged to the left eye of the person through the optical module 13, and the optical module 13 is configured to change a propagation direction of the light, so that the person can see the image displayed by the display component 11 through the left eye. Similarly, for the optical display module 2, when the display component 12 displays an image, light emitted by the display component 12 is converged to the right eye of the person through the optical module 14, so that the person can see the image displayed by the display component 12 through the right eye.

Types and quantities of display components 11 and display components 12 are not limited in embodiments of this application. Optionally, the display component 11 and the display component 12 may be two independent display components, or may be two display areas on a same display component. Optionally, the display component 11 and the display component 12 each may be a display, for example, a liquid crystal display, a light emitting diode (light emitting diode, LED) display, or another type of display component.

Optionally, the optical display module 1 and the optical display module 2 may be two lens tubes, and the lens tubes may be hollow and cylindrical. The display component 11, the optical module 13, the display component 12, and the optical module 14 are accommodated in the lens tubes. In this case, the display components and the optical modules are disposed on the VR glasses 100 through the lens tubes. In this embodiment of this application, the optical display module 1 and the optical display module 2 may also be referred to as lens tubes.

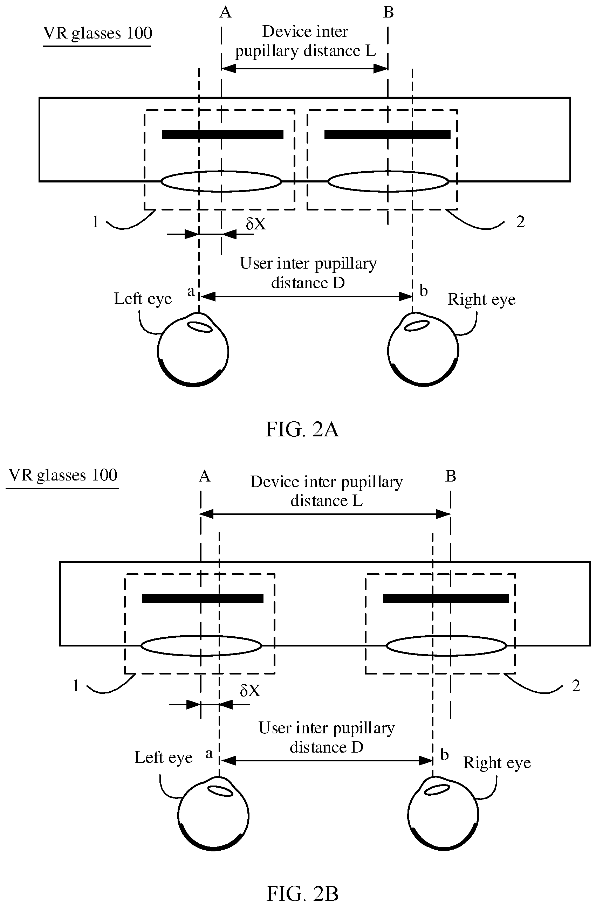

When the user wears a head-mounted display device, to obtain a good display effect and improve user experience, an inter pupillary distance of the user needs to match an inter pupillary distance of the head-mounted display device. The inter pupillary distance of the user is also referred to as a user inter pupillary distance or an inter pupillary distance of human eyes, and is a distance between pupils of both eyes of a person. The inter pupillary distance of the head-mounted display device is also referred to as a device inter pupillary distance, and is a distance between center lines of two optical modules on the head-mounted display device, for example, a distance between center lines of the optical module 13 and the optical module 14 in FIG. 1, or is a distance between center lines of two optical display modules, for example, a distance between center lines of the optical display module 1 and the optical display module 2 in FIG. 1. Different people usually have different inter pupillary distances. When the user wears the head-mounted display device, the device inter pupillary distance needs to be adjusted. For example, in a scenario, as shown in FIG. 2A, a device inter pupillary distance of the VR glasses 100 is a distance L between a reference line A and a reference line B (which may be understood as a distance between the optical display module 1 and the optical display module 2). The reference line A indicates a center line of the optical display module 1, and the reference line B indicates a center line of the optical display module 2. A user inter pupillary distance is a distance D between a reference line a and a reference line b. The reference line a corresponds to a pupil of a left eye of the user, and the reference line b corresponds to a pupil of a right eye of the user. In FIG. 2A, the device inter pupillary distance L is less than the user inter pupillary distance D, and the distance between the optical display module 1 and the optical display module 2 needs to be increased. For example, in another scenario, as shown in FIG. 2B, a device inter pupillary distance L is greater than a user inter pupillary distance D, and a distance between the optical display module 1 and the optical display module 2 needs to be decreased.

Currently, inter pupillary distance adjustment (IPD adjustment) of a device is generally implemented manually or mechanically. A component (for example, a dial knob) used by the user to manually adjust an inter pupillary distance is disposed on the head-mounted display device. The user turns the dial knob based on a viewing experience of the human eye during adjustment, to manually and mechanically adjust the inter pupillary distance of the head-mounted display device. An operation of the user is complex and complicated, adjustment precision is low, and an effect of inter pupillary distance adjustment is poor.

According to the inter pupillary distance adjustment method provided in embodiments of this application, after the user wears the head-mounted display device, the head-mounted display device may obtain inter pupillary distance adjustment information that indicates an inter pupillary distance adjustment value adjusted by the head-mounted display device, and may implement automatic adjustment on the inter pupillary distance of the head-mounted display device based on the inter pupillary distance adjustment information. This avoids manual and mechanical adjustment by the user, and improves convenience and accuracy of inter pupillary distance adjustment of the head-mounted display device.

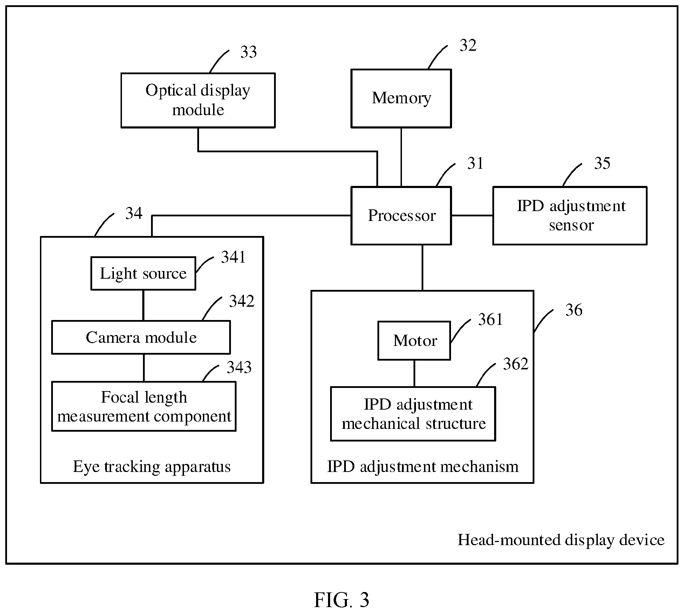

For example, FIG. 3 is a diagram of a structure of a head-mounted display device according to an embodiment of this application. As shown in FIG. 3, the head-mounted display device may include a processor 31, a memory 32, an optical display module 33, an eye tracking apparatus 34, an IPD adjustment sensor 35, and an IPD adjustment mechanism 36.

The processor 31 is configured to control an overall operation of the head-mounted display device, and may include one or more processing units. The processing units may be microcontroller units (microcontroller unit, MCU). For example, the processor 31 may include an application processor (application processor, AP), a modem processor, a graphics processing unit (graphics processing unit, GPU), an image signal processor (image signal processor, ISP), a video processing unit (video processing unit, VPU), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors.

A memory may be further disposed in the processor 31, and is configured to store instructions and data. In some embodiments, the memory in the processor 31 is a cache. The memory may store instructions or data just used or cyclically used by the processor 31. If the processor 31 needs to use the instructions or the data again, the processor may directly invoke the instructions or the data from the memory. This avoids repeated access, reduces waiting time of the processor 31, and improves system efficiency.

The processor 31 may include one or more interfaces. The interface may include an inter-integrated circuit (inter-integrated circuit, I2C) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, and/or a universal serial bus (universal serial bus, USB) interface, a serial peripheral interface (serial peripheral interface, SPI) interface, and the like.

In this embodiment of this application, the processor 31 may obtain related data of the eye tracking apparatus 34 and the IPD adjustment sensor 35, determine inter pupillary distance adjustment information, and control an operation of the IPD adjustment mechanism 36 based on the inter pupillary distance adjustment information, to adjust a distance between two optical display modules 33, thereby implementing automatic adjustment on an inter pupillary distance.

The memory 32 is configured to store executable program code and/or data, and the executable program code may include instructions. The processor 31 executes various functional applications of the head-mounted display device and data processing by running the instructions stored in the memory 32. The memory 32 may include a program storage area and a data storage area. The program storage area may store an operating system, an application required by at least one function (for example, a sound playing function or an image playing function), and the like. The data storage area may store data (such as audio data, image data, and video data) created during use of the head-mounted display device and the like. In addition, the memory 32 may include a high-speed random access memory, and may further include a non-volatile memory, for example, at least one disk storage device, a hard disk drive (hard disk drive, HDD), a solid-state drive (solid-state drive, SSD), a flash memory device, a universal flash storage (universal flash storage, UFS), or the like, or may include a volatile memory (volatile memory), for example, a random access memory (random access memory, RAM).

For a structure of the optical display module 33, refer to descriptions of the optical display module 1 and the optical display module 2 in FIG. 1. The optical display module 33 is configured to present an image to a user under control of the processor 31. The optical display module 33 may convert, by using one or more optical components of a reflector, a transmission mirror, an optical waveguide, or the like, real pixel image display into near-eye projected virtual image display, to implement virtual interaction experience or implement interaction experience combining virtual and reality. For example, the optical display module 33 receives image data information sent by the processor, and presents a corresponding image to the user. The processor 31 may control a focal power of the optical display module 33, to implement a function of adjusting a focal power of the head-mounted display device. For example, the processor 31 may adjust relative locations between optical components (such as a lens) in the optical display module 33, to adjust the focal power of the optical display module 33, so that a location of a corresponding virtual image surface may be adjusted when the optical display module 33 performs imaging on human eyes, thereby achieving an effect of controlling the focal power of the head-mounted display device.

The eye tracking apparatus 34 is configured to implement eye tracking on eyes of the user. The eye tracking apparatus 34 may determine a location of a fixation point of the user, or determine a direction of a line of sight of the user by using a method such as a video eye pattern method, a photodiode response method, or a pupil corneal reflection method. For example, according to the pupil corneal reflection method, a location of a pupil of a human eye is located, coordinates of a pupil center of the human eye are obtained, and a fixation point of the human eye is calculated, or an inter pupillary distance offset between the pupil of the human eye and a center line of a lens tube is calculated, to provide an accurate adjustment value for adjusting the inter pupillary distance of the head-mounted display device.

Optionally, the eye tracking apparatus 34 may include a light source 341, a camera module 342, and a focal length measurement component 343. A quantity and locations of light sources 341, a quantity and locations of camera modules 342, and a quantity and locations of focal length measurement components 343 are not limited in embodiments of this application. There may be at least one light source 341, at least one camera module 342, and at least one focal length measurement component 343. For example, the at least one light source 341 may be disposed on an end face that is of the head-mounted display device and that faces a human face (or eyes of the user), and the at least one camera module 342 may be further disposed on the end face that is of the head-mounted display device and that faces the human face (or the eyes of the user). The camera module 342 may photograph a light spot of the light source 341 reflected by a human eye, to implement eye tracking on the user. Optionally, the at least one light source 341 and the at least one camera module 342 may correspond to one optical display module 33, to implement eye tracking on one eye. Alternatively, both the at least one light source 341 and the at least one camera module 342 may correspond to two optical display modules 33, that is, a light source 341 and a camera module 342 are correspondingly disposed for each optical display module 33, to implement eye tracking on each eye. The focal length measurement component 343 is configured to detect focal length information of the optical display module 33. This may also be understood as detecting focal length information of an optical module in the optical display module 33.

A detection value of the IPD adjustment sensor 35 may reflect a current inter pupillary distance of the head-mounted display device, or reflect a location of the optical display module 33. A correspondence between the detection value and a device inter pupillary distance or a correspondence between the detection value and the location of the optical display module may be determined based on the detection value of the IPD adjustment sensor 35, and the correspondence may be referred to as a calibration table. A name and a type of the IPD adjustment sensor are not limited in embodiments of this application. For example, the IPD adjustment sensor may also be referred to as an IPD sensor or an inter pupillary distance sensor. The IPD adjustment sensor may include but is not limited to a Hall effect sensor or a slide rheostat, and the Hall effect sensor may also be referred to as a Hall device. When the IPD adjustment sensor is a Hall effect sensor, the calibration table may reflect a correspondence between a magnetic induction intensity detected by the Hall effect sensor and the device inter pupillary distance. When the IPD adjustment sensor is a slide rheostat, the calibration table may reflect a correspondence between a resistance value detected by the slide rheostat and the device inter pupillary distance.

The IPD adjustment mechanism 36 is configured to operate under control of the processor 31, to adjust a distance between two optical display modules 33, thereby implementing automatic adjustment on the inter pupillary distance of the head-mounted display device. Optionally, the IPD adjustment mechanism 36 may include a motor 361 and an IPD adjustment mechanical structure 362. The motor 361 is configured to drive the IPD adjustment mechanical structure 362 to operate. The IPD adjustment mechanical structure 362 is connected to the optical display module 33, and is configured to operate, under driving of the motor 361, to drive the two optical display modules 33 to move, and change a distance between the two optical display modules 33, thereby implementing device inter pupillary distance adjustment. A quantity of motors 361 is not limited in embodiments of this application. For example, there is one motor 361, and the motor 361 is configured to simultaneously drive the two optical display modules 33 to move; or there are two motors 361, and the two motors are configured to separately drive one optical display module 33 to move.

It may be understood that FIG. 3 does not constitute a limitation on a structure of the head-mounted display device. The head-mounted display device may include more or fewer components than those shown in FIG. 3, some components may be combined, some components may be split, or there may be different component arrangements. Optionally, the components shown in FIG. 3 may be implemented by using hardware, software, or a combination of software and hardware.

Optionally, the head-mounted display device may further include a battery, a microphone, a loudspeaker, a camera, a key, an input/output interface, a communication module, and the like.

The head-mounted display device may include one or more keys, and these keys may control the head-mounted display device to provide the user with access to functions on the head-mounted display device. The key may be in a form of a button, a switch, a dial, a touch sensor, or a near touch sensing device. For example, the user may turn on the optical display module 33 of the head-mounted display device by pressing a button. The keys may include a power button, a volume button, and the like. The keys may be mechanical keys, or may be touch keys. The head-mounted display device may receive key input, and generate key signal input related to user settings and function control of the head-mounted display device.

The input/output interface may connect another device to the head-mounted display device through an appropriate component. The component may include, for example, an audio/video jack, a data connector, and the like.

The communication module may include a wireless communication module and a mobile communication module, and is configured to communicate with another device. A communication function may be implemented by using an antenna, the mobile communication module, a modem processor, a baseband processor, or the like.

The antenna is configured to transmit and receive electromagnetic wave signals. The head-mounted display device may include a plurality of antennas, and each antenna may be configured to cover a single communication band or a plurality of communication bands. Different antennas may be further multiplexed, to improve antenna utilization.

The mobile communication module may provide a wireless communication solution applied to the head-mounted display device, and the wireless communication solution includes a solution for a 2nd generation (2nd generation, 2G) network, a 3rd generation (3rd generation, 3G) network, a 4th generation (4th generation, 4G) network, a 5th generation (5th generation, 5G) network, or the like.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to an application processor.

The wireless communication module may provide a wireless communication solution applied to the head-mounted display device, and the wireless communication solution includes a solution for a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module may be one or more components that integrate at least one communication processing module. The wireless communication module receives an electromagnetic wave through the antenna, performs frequency modulation and filtering processing on an electromagnetic wave signal, and sends a processed signal to the processor 31. The wireless communication module may further receive a to-be-sent signal from the processor 31, perform frequency modulation and amplification on the to-be-sent signal, and convert the to-be-sent signal into an electromagnetic wave for radiation through the antenna.

The following describes concepts in embodiments of this application and some structures and principles of a head-mounted display device with reference to FIG. 3.

1. Optical display module, optical module, and optical component

As shown in FIG. 1, FIG. 2A, and FIG. 2B, an optical display module is configured to display an image to an eye of a user, and a left eye and a right eye of the user respectively correspond to one optical display module. For example, in FIG. 1, the left eye of the user corresponds to the optical display module 1, and the right eye of the user corresponds to the optical display module 2.

The optical display module includes an optical module and a display component. The display component has a display function for displaying an image. The optical module includes at least one optical component. A quantity and a type of optical components are not limited in embodiments of this application. For example, the optical component may be a reflector, a transmission mirror, a Fresnel lens, an aspheric lens, an optical waveguide, or the like, or may be a lens group including a plurality of components. An optical path, a focal length, and a field of view vary with a quantity and a type of optical components included in the optical module.

Optionally, a location of the optical component in the optical module may be fixed, or the optical component may move in a direction away from or close to the display component, to change a focal power.

Optionally, the optical component in the optical module may be a fixed focal length lens, or may be a variable focal length lens.

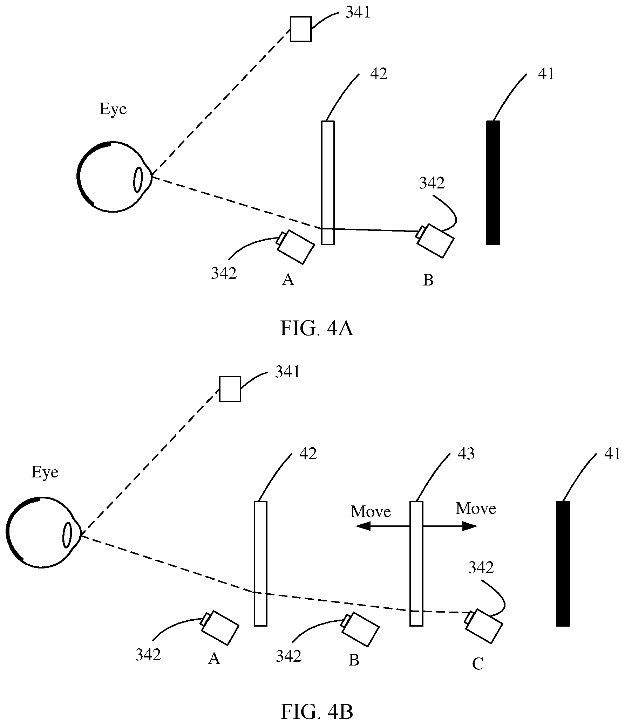

The following describes a structure of the optical display module with reference to FIG. 4A and FIG. 4B. However, FIG. 4A and FIG. 4B do not limit structures of the optical display module, the optical module, and the optical component. In FIG. 4A and FIG. 4B, a display component included in an optical display module is denoted as a display component 41.

In FIG. 4A, an optical module includes one optical component 42, and a location of the optical component 42 is fixed.

In FIG. 4B, an optical module includes two optical components: an optical component 42 and an optical component 43. A location of the optical component 42 is fixed, and the optical component 43 may move close to or away from the display component 41.

2. IPD adjustment mechanism and backlash error

According to the inter pupillary distance adjustment method provided in embodiments of this application, a processor may control an IPD adjustment mechanism to operate, to implement automatic adjustment on an inter pupillary distance of a head-mounted display device, and manual adjustment by a user is not required. When operation directions of the IPD adjustment mechanism are different, the inter pupillary distance of the head-mounted display device may be increased or decreased, that is, a distance between two optical display modules in the head-mounted display device may be increased or decreased. As shown in FIG. 3, the IPD adjustment mechanism 36 includes a motor 361 and an IPD adjustment mechanical structure 362. A name of the IPD adjustment mechanical structure 362 is not limited in embodiments of this application. For example, the IPD adjustment mechanical structure may also be referred to as a moving assembly. The motor 361 is connected to the IPD adjustment mechanical structure 362, and the IPD adjustment mechanical structure 362 is connected to the optical display module 33. The motor 361 moves under control of the processor, to drive the IPD adjustment mechanical structure 362 to operate. Further, the IPD adjustment mechanical structure 362 drives the two optical display modules 33 (or referred to as two lens tubes) in the head-mounted display device to move, to change a distance between the two optical display modules, thereby automatically adjusting the inter pupillary distance of the head-mounted display device.

Optionally, the IPD adjustment mechanical structure 362 may include a gear and a rack that cooperate with each other.

Optionally, in an implementation, the IPD adjustment mechanism 36 may control the two optical display modules to move simultaneously. This implementation is simple, requires few components, and occupies small space.

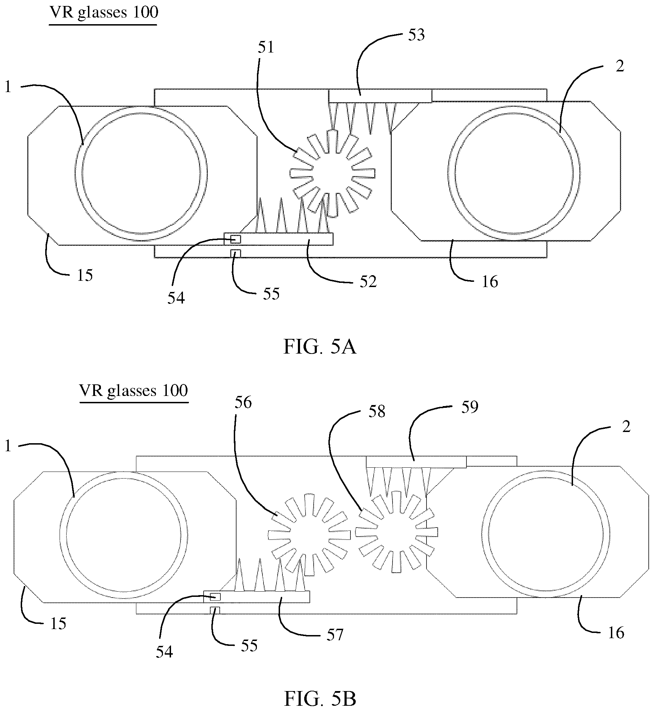

For example, as shown in FIG. 5A, VR glasses 100 include an optical display module 1 and an optical display module 2, the optical display module 1 is disposed in a lens tube 15, and the optical display module 2 is disposed in a lens tube 16. An IPD adjustment mechanism includes a motor (not shown) and an IPD adjustment mechanical structure, the IPD adjustment mechanical structure includes a gear 51 and two racks that cooperate with the gear 51, and the two racks are denoted as a rack 52 and a rack 53. The motor is connected to the gear 51, and the gear 51 is separately connected to the rack 52 and the rack 53. The rack 52 is connected to the optical display module 1 (or the lens tube 15), and is configured to drive the optical display module 1 to move. The rack 53 is connected to the optical display module 2 (or the lens tube 16), and is configured to drive the optical display module 2 to move.

When the motor moves under control of a processor, the motor drives the gear 51 to rotate. When the gear 51 rotates clockwise, the gear drives the rack 52 to move leftward, and drives the rack 53 to move rightward. The rack 52 drives the optical display module 1 to move leftward, and the rack 53 drives the optical display module 2 to move rightward. In this case, the optical display module 1 and the optical display module 2 move away from each other, a distance between the optical display module 1 and the optical display module 2 is gradually increased, and an inter pupillary distance of the head-mounted display device gradually is gradually increased. Similarly, when the gear 51 rotates counterclockwise, the rack 52 moves rightward, and the rack 53 moves leftward. In this case, a distance between the optical display module 1 and the optical display module 2 is gradually decreased, and an inter pupillary distance of the head-mounted display device is gradually decreased.

Optionally, in another implementation, the IPD adjustment mechanism 36 may separately control the two optical display modules to move. In this implementation, the two optical display modules may move separately, and inter pupillary distance adjustment is more flexibly.

For example, refer to FIG. 5B. A difference between FIG. 5B and FIG. 5A lies in that quantities of motors are different. Each motor corresponds to a respective IPD adjustment mechanical structure, and is configured to control movement of one optical display module. As shown in FIG. 5B, an IPD adjustment mechanism includes two motors (not shown, referred to as a motor A and a motor B). An IPD adjustment mechanical structure corresponding to the motor A includes a gear 56 and a rack 57, and an IPD adjustment mechanical structure corresponding to the motor B includes a gear 58 and a rack 59. The motor A is connected to the gear 56, the gear 56 is connected to the rack 57, and the rack 57 is connected to an optical display module 1 (or a lens tube 15), to drive the optical display module 1 to move. The motor B is connected to the gear 58, the gear 58 is connected to the rack 59, and the rack 59 is connected to an optical display module 2 (or a lens tube 16), to drive the optical display module 2 to move.

For the motor A, when the motor A moves under control of a processor, the motor A drives the gear 56 to rotate. When the gear 56 rotates clockwise, the gear 56 drives the rack 57 to move leftward, and further, the rack 57 drives the optical display module 1 to move leftward. When the gear 56 rotates counterclockwise, the gear 56 drives the rack 57 to move rightward, and the rack 57 drives the optical display module 1 to move rightward.

For the motor B, when the motor B moves under control of the processor, the motor B drives the gear 58 to rotate. When the gear 58 rotates clockwise, the gear 58 drives the rack 59 to move rightward, and further, the rack 59 drives the optical display module 2 to move rightward. When the gear 58 rotates counterclockwise, the gear 58 drives the rack 59 to move leftward, and the rack 59 drives the optical display module 2 to move leftward.

Optionally, when inter pupillary distance adjustment is performed, an inter pupillary distance adjustment direction may be a direction in which the optical display module 1 and the optical display module 2 move close to or move away from each other.

When the inter pupillary distance of the head-mounted display device is adjusted by using the IPD adjustment mechanical structure 362, because there is a gap between mechanical structures, when the motor moves in an opposite direction, the IPD adjustment mechanical structure cannot immediately move along with the motor in the opposite direction, and the IPD adjustment mechanical structure can move in the opposite direction only after the gap between the mechanical structures in the opposite direction is eliminated. This error may be referred to as a backlash error, an empty range error, or a hysteresis error. For example, as shown in FIG. 5A, the motor drives the gear 51 to rotate clockwise, and the gear 51 drives the rack 52 to move leftward. When the motor moves in an opposite direction, the motor drives the gear 51 to change from clockwise rotation to counterclockwise rotation. Because there is a gap between the gear 51 and teeth on the rack 52, the rack 52 cannot immediately change from moving leftward to moving rightward. Specifically, after the gear 51 is driven to rotate counterclockwise for first duration by displacement of the motor in an opposite direction, the gap between the gear 51 and the teeth on the rack 52 is eliminated. Then, the motor continues to drive the gear 51 to rotate counterclockwise, and the gear 51 may drive the rack 52 to move rightward.

In embodiments of this application, to improve accuracy of inter pupillary distance adjustment of the head-mounted display device, when inter pupillary distance adjustment directions are opposite, influence of the backlash error needs to be considered.

It should be noted that a value of the backlash error is not limited in embodiments of this application. Backlash errors of different mechanical structures are different, and may be values measured by using an instrument, or may be empirical values.

3. Eye tracking apparatus and inter pupillary distance offset of a single eye

In embodiments of this application, if a head-mounted display device includes an eye tracking apparatus, eye tracking may be performed on a fixation point of a human eye of a user by using the eye tracking apparatus, to obtain an inter pupillary distance offset of a single eye. The inter pupillary distance offset of the single eye indicates a deviation between a pupil of the single eye and a corresponding optical display module. For example, as shown in FIG. 2A or FIG. 2B, the inter pupillary distance offset of the single eye may be represented as $\delta X$. Optionally, a value of the inter pupillary distance offset $\delta X$ may be a positive value, or may be a negative value, and whether a current device inter pupillary distance of the head-mounted display device is greater than or less than an inter pupillary distance of human eyes may be determined by using a positive value or a negative value of the inter pupillary distance offset $\delta X$. For example, if a value of an inter pupillary distance offset $\delta X$ in FIG. 2A is positive, a value of an inter pupillary distance offset $\delta X$ in FIG. 2B is negative. Alternatively, if a value of an inter pupillary distance offset $\delta X$ in FIG. 2A is negative, a value of an inter pupillary distance offset $\delta X$ in FIG. 2B is positive.

It can be learned that the head-mounted display device may automatically obtain an inter pupillary distance measurement value that needs to be adjusted by using the eye tracking apparatus. Compared with a case in which a user manually adjusts an inter pupillary distance of a device based on a feeling of eyes of the user, accuracy of obtaining the inter pupillary distance adjustment value is improved, and accuracy of performing inter pupillary distance adjustment by the head-mounted display device is improved.

In addition, the eye tracking apparatus may further continue to obtain the inter pupillary distance offset of the single eye in real time, and determine, based on the inter pupillary distance offset of the single eye, whether inter pupillary distance adjustment of the device can be ended. For example, as shown in FIG. 2B, a device inter pupillary distance L is greater than a user inter pupillary distance D, and the optical display module 1 and the optical display module 2 need to be moved close to each other, to decrease the device inter pupillary distance. In a process of reducing the device inter pupillary distance, the inter pupillary distance offset $\delta X$ of the single eye may be obtained in real time by using the eye tracking apparatus. If the inter pupillary distance offset $\delta X$ of the single eye falls within a first error range, it may be determined that inter pupillary distance adjustment ends, and automatic inter pupillary distance adjustment is completed. Otherwise, if the inter pupillary distance offset $\delta X$ of the single eye exceeds the first error range, the device inter pupillary distance continues to be adjusted. A value of the first error range is not limited in embodiments of this application.

It can be learned that the inter pupillary distance offset obtained by using the eye tracking apparatus provides an accurate basis for determining whether to end inter pupillary distance adjustment, thereby improving accuracy of performing inter pupillary distance adjustment by the head-mounted display device.

In terms of structure, an eye tracking apparatus may be correspondingly disposed in one of two optical display modules of the head-mounted display device, or an eye tracking apparatus may be disposed in each optical display module.

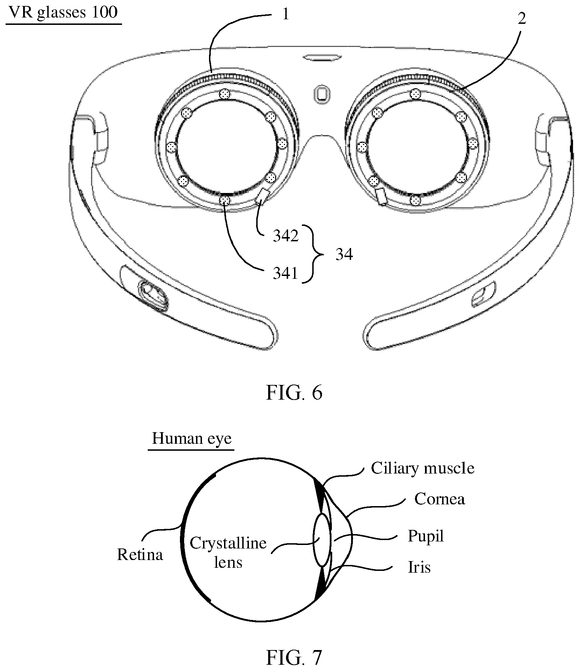

For example, an example in which an eye tracking apparatus is disposed in each of the two optical display modules is used for description. FIG. 6 is a diagram of a structure of an eye tracking apparatus according to an embodiment of this application. As shown in FIG. 6, VR glasses 100 include an optical display module 1 and an optical display module 2. The optical display module 1 and the optical display module 2 respectively correspond to an eye tracking apparatus 34. The eye tracking apparatus 34 includes at least one light source 341 and a camera module 342. A quantity, a type, and distribution locations of light sources 341 are not limited in embodiments of this application, and a type, a structure, and a disposition location of the camera module 342 are not limited in embodiments of this application. For example, in FIG. 6, there are eight light sources 341 corresponding to the optical display module 1, and the light sources 341 are evenly distributed in a circular shape on an eye-facing end surface of the optical display module 1, and the camera module 342 is disposed on the eye-facing end surface of the optical display module 1. For example, the light source 341 may be a near infrared light emitting diode (light emitting diode, LED), and the camera module 342 may be a near infrared camera.

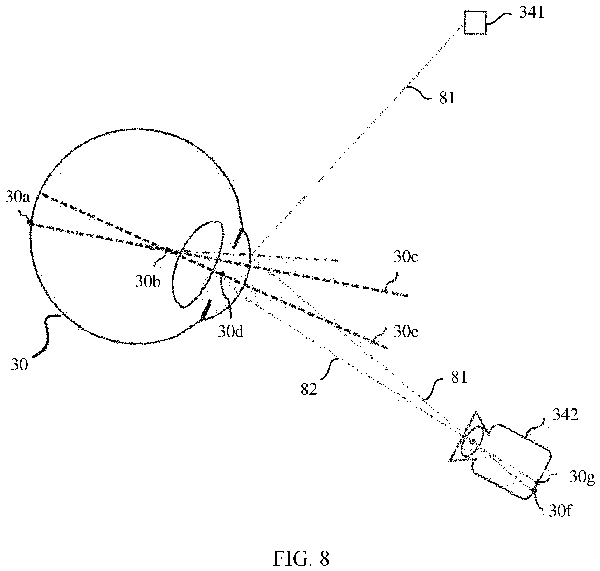

With reference to FIG. 7 and FIG. 8, the following describes, by using an example in which a pupil corneal reflection method is used on a single eye, a principle of implementing eye tracking by an eye tracking apparatus to obtain an inter pupillary distance offset of the single eye.

For example, FIG. 7 is a diagram of composition of a human eye according to an embodiment of this application. As shown in FIG. 7, the human eye may include a retina, a crystalline lens, a ciliary muscle, a pupil, an iris, and a cornea. The cornea is a circular transparent part in the front of an outer layer of an eyeball wall, the iris is a flat circular annular thin film in a middle layer of the eyeball wall, and the pupil is a small circular hole in the center of the iris in the human eye, and is a channel through which light enters the eye. The crystalline lens is located behind the iris, and the crystalline lens may function as a zoom lens to perform convergence processing on light emitted into the human eye, and converge incident light onto the retina at the bottom of the human eye, to form a clear image of a scene in an actual scenario on the retina. The ciliary muscle may adjust a form of the crystalline lens. For example, the ciliary muscle may adjust a diopter of the crystalline lens by shrinking or relaxing the ciliary muscle, to adjust a focal length of the crystalline lens. In this way, objects at different distances in an actual scenario can be clearly imaged on the retina through the crystalline lens.

For example, FIG. 8 is a diagram of a principle of corneal reflection-based eye tracking according to an embodiment of this application. A light source 341 in FIG. 8 may be any light source 341 in FIG. 6, and a camera module 342 in FIG. 8 may be the camera module 342 in FIG. 6. As shown in FIG. 8, a connection line between a foveal 30a of an eyeball 30 of a user and a corneal sphere center 30b is a visual axis 30c of the eyeball 30, that is, a line-of-sight direction. A connection line between a pupil center 30d and the corneal sphere center 30b is an optical axis 30e of the eyeball 30, that is, an optical axis center of the eyeball 30. There is an included angle between the optical axis 30e and the visual axis 30c of the eyeball 30, and some detection results are that the included angle is 5°.

When the eye tracking apparatus uses a pupil corneal reflection method, light emitted by the light source 341 enters the camera module 342 after being reflected at a cornea of the eyeball 30. Refer to a dashed line 81. The camera module 342 may obtain, through photographing, a light spot 30f corresponding to the light source 341. In addition, refer to a dashed line 82. The pupil of the eyeball 30 is refracted at the cornea to the camera module 342, and the camera module 342 may obtain, through photographing, a light spot 30g corresponding to the pupil of the eyeball 30, and find the pupil center 30d based on the light spot 30g. A normal determined based on parameters such as a location of the light source 341, a location of the camera module 342, a radius R of the cornea, a distance K from the pupil to a vertex of the cornea, and a refractive index n of the cornea and according to a reflection law passes through the corneal sphere center 30b, a location of the corneal sphere center 30b may be calculated based on a location of the light spot 30f of the light source 341, and then the optical axis 30e of the eyeball 30 and coordinates P of the pupil may be obtained based on a location of an image 30g of the pupil center. Generally, a plurality of light sources 341 are evenly distributed around a center line of an optical display module, that is, the center line of the optical display module is located at a center O of a circle formed by the plurality of light sources 341. Therefore, a distance between two points of O and P, that is, the inter pupillary distance offset $\delta X$ of the single eye, may be calculated based on the coordinates P of the pupil and coordinates O of the circle center. Related parameters may be represented as follows: A quantity of light sources 341 is j, three-dimensional coordinates of the j light sources 341 may be represented as lj(xlj, ylj, zlj), where j=0, 1, 2..., three-dimensional coordinates of the camera module 342 may be represented as C(xC, yC, zC), the three-dimensional coordinates P of the pupil may be represented as P(xP, yP, zP), and the three-dimensional coordinates of the circle center O may be represented as O(xO, yO, zO). x, y, and z respectively represent an x-axis, a y-axis, and a z-axis in a three-dimensional coordinate system.

Optionally, in some embodiments, the visual axis 30c of the eyeball 30 may be further obtained through calibration.

In embodiments of this application, an optical module in an optical display module may include at least one optical component. When implementations of the optical module are different, the camera module 342 may be disposed at different locations. Different locations of the camera module 342 affect optical path calculation of the camera module 342, and an axial aberration and/or a vertical aberration introduced by refraction of light by an optical component in the optical module need to be considered.

Optionally, in an implementation, the camera module 342 may be located between an optical module and a human eye. In this case, the camera module 342 is located in front of all optical components in the optical module and is close to the human eye, and the camera module 342 is outside an optical system. For example, refer to a camera module 342 at a location A in FIG. 4A or FIG. 4B. In this case, for an optical path principle, refer to FIG. 8.

Optionally, in another implementation, the camera module 342 may be located between two adjacent optical components in an optical module. In this case, the camera module 342 is in an optical system, and refraction of an optical component located in front of the camera module 342 needs to be considered during optical path calculation. For example, refer to a camera module 342 at a location B in FIG. 4B. In this case, influence of refraction of the optical component 42 on an optical path needs to be considered.

Optionally, in still another implementation, the camera module 342 may be located between an optical module and a display component. In this case, the camera module 342 is located behind all optical components in the optical module and is close to the display component, the camera module 342 is in an optical system, and refraction of all the optical components in the optical module needs to be considered during optical path calculation. For example, refer to a camera module 342 at a location B in FIG. 4A. In this case, influence of refraction of the optical component 42 on an optical path needs to be considered. A dashed line in FIG. 4A shows an optical path in which light emitted by the light source 341 enters the camera module 342 at the location B after being reflected at a cornea of a human eye. For another example, refer to a camera module 342 at a location C in FIG. 4B. In this case, influence of refraction of an optical component 42 and an optical component 43 on an optical path needs to be considered. For example, a dashed line in FIG. 4B shows an optical path in which light emitted by the light source 341 enters the camera module 342 at the location C after being reflected at a cornea of a human eye.

Optionally, an optical component in an optical module may be moved, to change a focal length of the optical module. For example, refer to the optical component 43 in FIG. 4B. The eye tracking apparatus may further include a focal length measurement component that is configured to detect focal length information of an optical module. Optionally, the focal length measurement component may include but is not limited to a Hall effect sensor, a nearsighted adjustment Hall effect sensor, an infrared laser ranging unit, or a slide rheostat.

Optionally, the eye tracking apparatus may further include a light reflective component that is configured to change a direction of light in an optical path through reflection, so that the light enters the camera module 342 after being reflected. A type and a disposing location of the light reflective component are not limited in embodiments of this application. For example, the light reflective component is an infrared reflector.

4. IPD adjustment sensor and calibration table

A detection value of an IPD adjustment sensor may reflect a current location of an optical display module, or reflect a current inter pupillary distance of a head-mounted display device. A correspondence between the detection value and a device inter pupillary distance may be obtained based on the detection value of the IPD adjustment sensor, and the correspondence may be referred to as a calibration table.

The following describes an implementation and a detection principle of the IPD adjustment sensor by using an example in which the IPD adjustment sensor is a Hall effect sensor.

For example, as shown in FIG. 5A, an IPD adjustment sensor includes a magnet 54 and a Hall effect sensor 55. A magnetic induction intensity (which may be understood as a magnetic flux) detected by the Hall effect sensor 55 may be obtained through magnetic induction between the magnet and the Hall effect sensor, and is denoted as B(Bx, By, Bz), where Bx, By, and Bz respectively represent spatial components on three axes x, y, and z. In FIG. 5A, the Hall effect sensor 55 is disposed on a printed circuit board (printed circuit board, PCB), and a location of the Hall effect sensor 55 remains unchanged. The magnet 54 is disposed on the rack 52 in the IPD adjustment mechanism. The rack 52 may move leftward or rightward under driving of the motor, to drive the optical display module 1 to move leftward or rightward, and adjust a device inter pupillary distance of the VR glasses 100. When the rack 52 moves, the rack 52 drives the magnet 54 to move, a location between the magnet 54 and the Hall effect sensor 55 changes, and the magnetic induction intensity B(Bx, By, Bz) detected by the Hall effect sensor 55 changes.

It can be learned that the inter pupillary distance of the VR glasses 100 is changed by using the IPD adjustment mechanism, so that the Hall effect sensor 55 can continuously detect the magnetic induction intensity when the inter pupillary distance of the VR glasses 100 changes. A correspondence between the magnetic induction intensity and a location of the optical display module may be established based on the magnetic induction intensity B(Bx, By, Bz) detected by the Hall effect sensor 55 and spatial coordinates (x, y, z) of the optical display module 1, to form a calibration table.

In some implementations, the head-mounted display device may locally store a calibration table in advance.

In some implementations, the head-mounted display device may communicate with a cloud server, obtain a calibration table from the cloud server, and locally store the calibration table.

In an implementation of determining a calibration table, an industrial camera (for example, a telecentric lens camera) may be used to capture a lens tube feature, including but not limited to a lens skirt edge, a frosted atomization surface, an outer diameter of a lens tube mechanical part, a feature of the lens tube mechanical part, and the like, to capture a real-time IPD distance L between dual lens tubes, and a processor reads a magnetic induction intensity reported by the Hall effect sensor, to obtain a correspondence between the magnetic induction intensity B(Bx, By, Bz) and the IPD distance L, and obtain the calibration table. A presentation form of the calibration table is not limited in embodiments of this application, and the calibration table may be a data table, or may be a curve function. Optionally, a calibration curve of the magnetic induction intensity B and the IPD distance L may be obtained through fitting. Optionally, based on a relative placement relationship between the magnet and the Hall effect sensor, an axis component with a largest change slope may be used as a basis for determining intensity distribution, and a symmetric component is selected as a basis for determining direction distribution, to ensure uniqueness of reverse solution of the IPD distance by using the magnetic induction intensity.

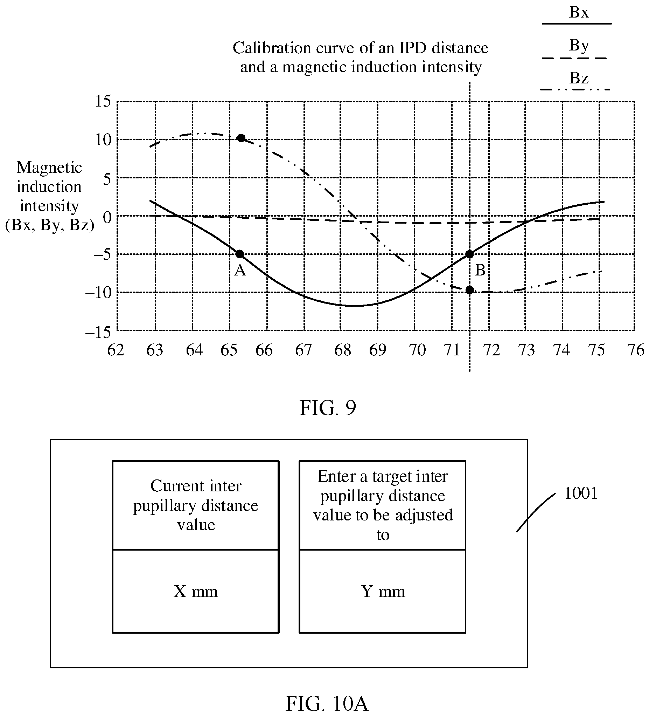

For example, FIG. 9 is a diagram of a calibration curve of an IPD distance and a magnetic induction intensity according to an embodiment of this application. As shown in FIG. 9, a horizontal axis represents an IPD distance in millimeters (mm), and a vertical axis represents the magnetic induction intensity B(Bx, By, Bz). Between three axial components (Bx, By, Bz) of the magnetic induction intensity B, a change slope of an axial component Bx is the largest, and the axial component Bx is used as a basis for determining intensity distribution. Then, an axial component Bz is selected as a basis for determining direction distribution. For example, when the axis component Bx is -5, there are two points on a curve of the axis component Bx, which are respectively a point A and a point B. Then, the IPD distance is finally determined based on the axis component Bz. It is assumed that the axis component Bz is a negative value, it may be determined that a current IPD distance is 71.5 millimeters, thereby ensuring that a unique value of the IPD distance can be resolved by using the magnetic induction intensity.

Optionally, when the calibration curve of the magnetic induction intensity B and the IPD distance L is fitted, to ensure fitting precision, a fitting polynomial may be determined based on collected point data. For example, the fitting polynomial is a higher-order polynomial $Bx=AL^X+BL^{X-1}+...+CONS$, where A and B represent calibrated coefficients, L represents an IPD distance, and CONS represents a constant; or the fitting polynomial is a fitting polynomial including a trigonometric function.

Optionally, in embodiments of this application, stepless continuous adjustment or limited level precision adjustment may be implemented during inter pupillary distance adjustment of the head-mounted display device. For example, an adjustable range of the inter pupillary distance of the head-mounted display device is 58 mm to 72 mm, and 0.2 mm may be adjusted in each level (a unit level). In a manner of limited level precision adjustment, it is required that the magnetic induction intensity detected by the Hall effect sensor meets two conditions: (1) A magnetic induction intensity at any point is less than a measurement range of the Hall effect sensor; and (2) precision of each level is greater than Hall effect sensor sensitivity.

Optionally, because a backlash error may be introduced when a device inter pupillary distance is adjusted by using the IPD adjustment mechanical structure, to improve accuracy of the calibration table and accuracy of adjusting the inter pupillary distance of the device, when the Hall effect sensor continuously detects the magnetic induction intensity, the Hall effect sensor may be moved from one inter pupillary distance limit location to another inter pupillary distance limit location of the head-mounted display device. For example, an adjustable range of the inter pupillary distance of the head-mounted display device is 58 mm to 72 mm, and the Hall effect sensor may be moved from a minimum IPD limit location of 58 mm to a maximum IPD limit location of 72 mm, or may be moved from a maximum IPD limit location of 72 mm to a minimum IPD limit location of 58 mm. In this way, different inter pupillary distance adjustment directions correspond to one calibration table. When the calibration table is subsequently used, corresponding calibration tables may be used based on different inter pupillary distance adjustment directions.

Locations of the magnet 54 and the Hall effect sensor 55 are not limited in embodiments of this application. For example, in FIG. 5A, the magnet 54 may be disposed on the PCB, and the Hall effect sensor 55 is disposed on the rack 52.

A type of the IPD adjustment sensor is not limited in embodiments of this application, and detection values vary with types of the IPD adjustment sensor. For example, the IPD adjustment sensor may be a slide rheostat. In this case, a detection value may be a resistance value, and the calibration table may reflect a correspondence between the resistance value and a location of an optical display module (or a device inter pupillary distance).

Optionally, an IPD adjustment sensor may be correspondingly disposed in one of two optical display modules in the head-mounted display device, or an IPD adjustment sensor may be correspondingly disposed in each of the two optical display modules.

In embodiments of this application, if the head-mounted display device includes an IPD adjustment sensor, the IPD adjustment sensor may obtain a detection value in a current device inter pupillary distance in real time, and may determine the current inter pupillary distance of the head-mounted display device according to the calibration table. In a process in which the head-mounted display device automatically adjusts the inter pupillary distance, whether inter pupillary distance adjustment of the device can be ended may be determined based on the detection value of the IPD adjustment sensor.

Optionally, in an implementation, a target detection value is obtained, and the target detection value is a detection value corresponding to a target inter pupillary distance (or a target inter pupillary distance value) of the head-mounted display device. A current detection value is obtained in real time, and if a difference between the current detection value and the target detection value falls within a first error range, it is determined that inter pupillary distance adjustment of the device ends, and automatic adjustment on the inter pupillary distance is completed. Otherwise, if a difference between the current detection value and the target detection value exceeds the first error range, the inter pupillary distance of the device continues to be adjusted.

Optionally, in another implementation, a target inter pupillary distance (or a target inter pupillary distance value) of the head-mounted display device is obtained. A current detection value of the IPD adjustment sensor is obtained in real time, and a current inter pupillary distance (or a current inter pupillary distance value) corresponding to the current detection value is obtained according to the calibration table. If a difference between the current inter pupillary distance value and the target inter pupillary distance value falls within a first error range, it is determined that inter pupillary distance adjustment of the device ends, and automatic adjustment on the inter pupillary distance is completed. Otherwise, if a difference between the current inter pupillary distance value and the target inter pupillary distance value exceeds the first error range, the inter pupillary distance of the device continues to be adjusted.

It should be noted that a value range of the first error range is not limited in embodiments of this application.

It can be learned that the IPD adjustment sensor and the calibration table provide an accurate basis for ending inter pupillary distance adjustment, thereby improving accuracy of performing inter pupillary distance adjustment by the head-mounted display device.

The following describes an example of a display interface in embodiments of this application.

According to the inter pupillary distance adjustment method provided in embodiments of this application, inter pupillary distance adjustment information may be obtained by using an eye tracking apparatus, or the inter pupillary distance adjustment information may be entered by a user, to obtain the inter pupillary distance adjustment information.

Optionally, in an implementation, the user may enter a target inter pupillary distance value that needs to be adjusted to. For example, as shown in FIG. 10A, a head-mounted display device displays an interface 1001. The interface 1001 includes a current inter pupillary distance value X mm of the head-mounted display device, and further guides, through prompt information, the user to enter the target inter pupillary distance value. For example, the prompt information is a text "Please enter a target inter pupillary distance value that you want to adjust to". For example, the user may enter a value Y in millimeters.

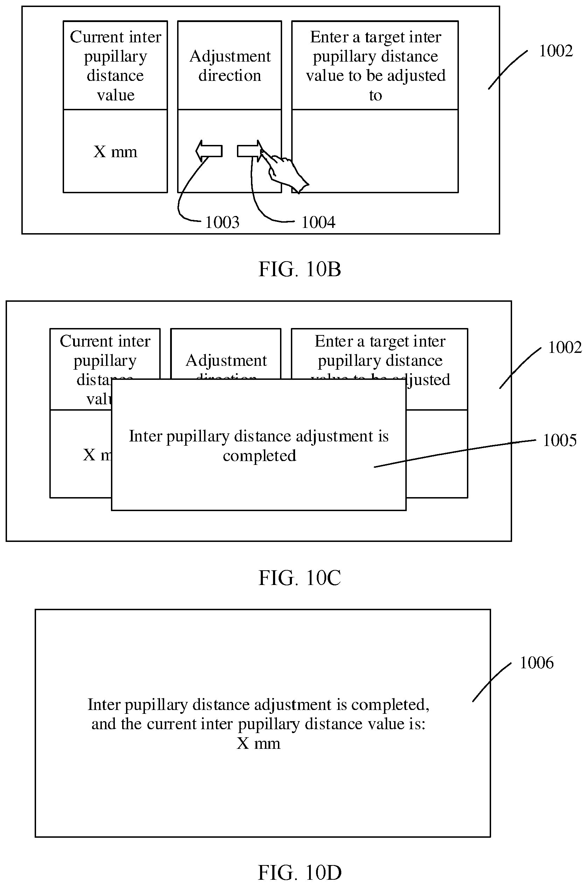

Optionally, in another implementation, the user may enter an inter pupillary distance adjustment direction. Correspondingly, the head-mounted display device may adjust the device inter pupillary distance based on the adjustment direction entered by the user and preset unit levels. An inter pupillary distance adjusted in one level is not limited in embodiments of this application. For example, 0.2 mm to 1 mm may be adjusted in each level. For example, as shown in FIG. 10B, a head-mounted display device displays an interface 1002. The interface 1002 includes a current inter pupillary distance value X mm of the head-mounted display device, and guides, through prompt information, the user to enter an inter pupillary distance adjustment direction or a target inter pupillary distance value. For example, the prompt information is a text "Adjustment direction", an arrow control 1003 and an arrow control 1004, and a text "Please enter a target inter pupillary distance value that you want to adjust to". It is assumed that the user taps the arrow control 1004, to indicate that an inter pupillary distance adjustment direction is a direction in which the inter pupillary distance is increased and the two optical display modules move away from each other.

According to the inter pupillary distance adjustment method provided in this embodiment of this application, the inter pupillary distance of the head-mounted display device can be automatically adjusted. After the inter pupillary distance adjustment is completed, prompt information may be output, to inform the user that inter pupillary distance adjustment is ended. Optionally, the prompt information may include an adjusted device inter pupillary distance value.

For example, in an example, as shown in FIG. 10C, the head-mounted display device displays the interface 1002. After the inter pupillary distance adjustment is completed, a window 1005 may pop up in the interface 1002. The window 1005 includes the prompt information. For example, the prompt information is a text "Inter pupillary distance adjustment is completed".

For example, in another example, as shown in FIG. 10D, a head-mounted display device displays an interface 1006, and the interface 1006 includes prompt information. For example, the prompt information is a text "Inter pupillary distance adjustment is completed, and a current inter pupillary distance value is: Y mm".

It should be noted that FIG. 10A to FIG. 10D constitute no limitation on a related interface, and a layout and display content of the interface may be adjusted based on requirements. Optionally, the related interface may be an interface of a system application, or an interface of a third-party application (application, APP).

The following describes in detail technical solutions of this application by using specific embodiments. The following embodiments may be combined with each other, and same or similar concepts or processes may not be repeatedly described in some embodiments.

Terms "first", "second", "third", "fourth", and the like (if any) in embodiments of this application are used to distinguish between similar objects, but not necessarily describe a specific order or sequence.

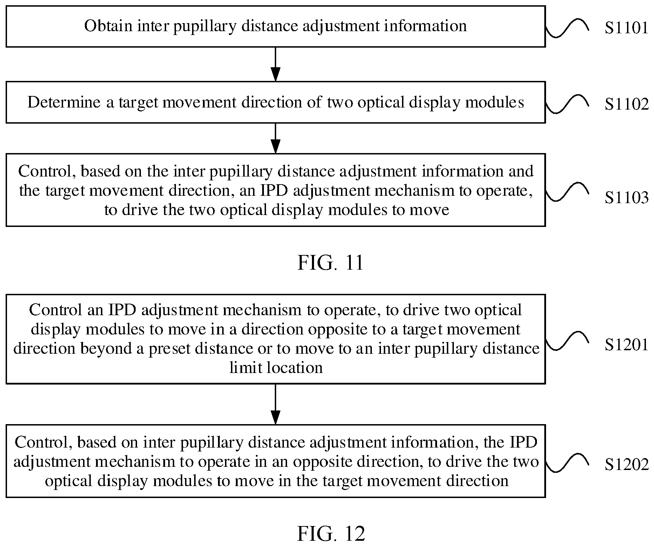

FIG. 11 is a flowchart of an inter pupillary distance adjustment method according to an embodiment of this application. The inter pupillary distance adjustment method provided in this embodiment is executed by a head-mounted display device. As shown in FIG. 11, the inter pupillary distance adjustment method provided in this embodiment may include the following steps.

S1101: Obtain inter pupillary distance adjustment information.

The inter pupillary distance adjustment information represents an inter pupillary distance adjustment value adjusted by the head-mounted display device, and the head-mounted display device may adjust an inter pupillary distance of the device based on the inter pupillary distance adjustment information.

Optionally, if the head-mounted display device includes an eye tracking apparatus, obtaining the inter pupillary distance adjustment information may include: obtaining an inter pupillary distance offset of a single eye by using the eye tracking apparatus. The inter pupillary distance offset of the single eye indicates a deviation between a pupil of the single eye and a corresponding optical display module.

For example, as shown in FIG. 2A or FIG. 2B, the inter pupillary distance offset of the single eye may be represented as $\delta X$. For a principle of the eye tracking apparatus, refer to the foregoing descriptions in this application. Details are not described herein again. In this implementation, the inter pupillary distance adjustment information includes the inter pupillary distance offset of the single eye, and the inter pupillary distance adjustment value that needs to be adjusted by the head-mounted display device may be automatically obtained by using the eye tracking apparatus. This improves accuracy of obtaining the inter pupillary distance adjustment information. In addition, compared with adjusting an inter pupillary distance based on a viewing feeling of a human eye of a user, this manner improves accuracy of performing inter pupillary distance adjustment by the head-mounted display device.

In this embodiment, the head-mounted display device includes two optical display modules, and an eye tracking apparatus may be disposed in one of the two optical display modules, or an eye tracking apparatus may be disposed in each optical display module.

Optionally, if an eye tracking apparatus is disposed in only one optical display module, or an eye tracking apparatus is disposed in each optical display module, obtaining the inter pupillary distance offset of the single eye by using the eye tracking apparatus may include: performing eye tracking on a left eye or a right eye of a person by using the eye tracking apparatus, and determining an obtained inter pupillary distance offset of the left eye or an obtained inter pupillary distance offset of the right eye as the inter pupillary distance offset of the single eye.

In this implementation, there is only one inter pupillary distance offset of the single eye, which is specifically the inter pupillary distance offset of the left eye or the inter pupillary distance offset of the right eye. Because structures of human eyes and the head-mounted display device are usually symmetrical, performing eye tracking on a single eye to obtain an inter pupillary distance offset of the single eye is simple to implement.

Optionally, if an eye tracking apparatus is disposed in each optical display module, obtaining the inter pupillary distance offset of the single eye by using the eye tracking apparatus may include:

separately performing eye tracking on a left eye and a right eye of a person by using the eye tracking apparatus, and determining the inter pupillary distance offset of the single eye based on an obtained inter pupillary distance offset of the left eye and an obtained inter pupillary distance offset of the right eye.

In this implementation, eye tracking is performed on each eye to obtain inter pupillary distance offsets respectively corresponding to the left eye and the right eye, and the inter pupillary distance offset of the single eye is finally determined based on the inter pupillary distance offset of the left eye and the inter pupillary distance offset of the right eye. This improves accuracy of determining the inter pupillary distance offset.

Optionally, in an implementation, determining the inter pupillary distance offset of the single eye based on the obtained inter pupillary distance offset of the left eye and the obtained inter pupillary distance offset of the right eye may include:

determining the inter pupillary distance offset of the left eye and the inter pupillary distance offset of the right eye as the inter pupillary distance offset of the single eye.

In this implementation, there are two inter pupillary distance offsets of the single eye that include the inter pupillary distance offset of the left eye and the inter pupillary distance offset of the right eye. Subsequently, in a process of adjusting the inter pupillary distance of the head-mounted display device, locations of optical display modules corresponding to the left eye and the right eye may be separately moved. This improves accuracy and flexibility of inter pupillary distance adjustment of the device.

Optionally, in another implementation, determining the inter pupillary distance offset of the single eye based on the obtained inter pupillary distance offset of the left eye and the obtained inter pupillary distance offset of the right eye may include:

determining an average value of the inter pupillary distance offset of the left eye and the inter pupillary distance offset of the right eye as the inter pupillary distance offset of the single eye.

In this implementation, there is one inter pupillary distance offset of the single eye. An average value is calculated. This improves accuracy of determining the inter pupillary distance offset of the single eye.

Optionally, if the head-mounted display device includes the eye tracking apparatus, and further includes an IPD adjustment sensor, obtaining the inter pupillary distance adjustment information may further include:

> obtaining a current detection value of the IPD adjustment sensor;
> obtaining a calibration table, where the calibration table indicates a correspondence between a detection value of the IPD adjustment sensor and an inter pupillary distance of the head-mounted display device; and
> determining a target inter pupillary distance value and/or a target detection value based on the calibration table, the inter pupillary distance offset of the single eye, and the current detection value.

The following provides descriptions with reference to examples. It is assumed that the IPD adjustment sensor is a Hall effect sensor, and the detection value is a magnetic induction intensity. The current detection value of the IPD adjustment sensor is a magnetic induction intensity B 1, and a device inter pupillary distance IPD1 corresponding to the magnetic induction intensity B 1 may be obtained according to the calibration table, that is, a current inter pupillary distance value of the head-mounted display device is IPD1. It is assumed that the inter pupillary distance offset of the single eye is $\delta X$. In this case, the target inter pupillary distance value meets: IPD2=IPD1+2$\delta$x. A target detection value B2 corresponding to a target inter pupillary distance value IPD2 may be obtained according to the calibration table.

In this implementation, the inter pupillary distance adjustment information includes the target inter pupillary distance value and/or the target detection value, and an inter pupillary distance adjustment value may be obtained by using the IPD adjustment sensor and the calibration table. In addition, in a process of adjusting the device inter pupillary distance, whether inter pupillary distance adjustment can be ended may be determined based on the target inter pupillary distance value and/or the target detection value, and the current inter pupillary distance value and/or the current detection value. This improves accuracy of inter pupillary distance adjustment.

Optionally, if different inter pupillary distance adjustment directions respectively correspond to calibration tables, when an inter pupillary distance adjustment direction is obtained, a calibration table corresponding to the inter pupillary distance adjustment direction needs to be used.

Optionally, the inter pupillary distance adjustment information may be entered by the user. A manner in which the user enters the inter pupillary distance adjustment information is not limited in this embodiment. For example, the inter pupillary distance adjustment information may be entered through voice, or may be entered by performing an operation on an interface displayed on the head-mounted display device.

Optionally, in an implementation, obtaining the inter pupillary distance adjustment information may include: obtaining a target inter pupillary distance value entered by the user.

In this implementation, the inter pupillary distance adjustment information includes the target inter pupillary distance value entered by the user. This is applicable to a scenario in which the user learns of an inter pupillary distance of human eyes.

Optionally, obtaining the target inter pupillary distance value entered by the user may include: obtaining the target inter pupillary distance value entered by the user through voice; or displaying a first interface, and obtaining the target inter pupillary distance value entered by the user in the first interface. For example, for the first interface, refer to FIG. 10B or FIG. 10C.

Optionally, in another implementation, obtaining the inter pupillary distance adjustment information may include: obtaining an inter pupillary distance adjustment direction entered by the user.

In this implementation, the inter pupillary distance adjustment information includes the interpupillary distance adjustment direction entered by the user, and the inter pupillary distance adjustment direction may include a direction in which the inter pupillary distance is increased and the two optical display modules move away from each other or a direction in which the inter pupillary distance is decreased and the two optical display modules move close to each other. Subsequently, the head-mounted display device may adjust the device inter pupillary distance according to a preset unit level. An inter pupillary distance adjusted in one level is not limited in embodiments of this application. For example, 0.2 mm to 1 mm may be adjusted in each level. For example, as shown in FIG. 10B, each time the user taps the arrow control 1004, the device inter pupillary distance may be increased by 0.5 mm, and each time the user taps the arrow control 1003, the device inter pupillary distance may be decreased by 0.5 mm.

S1102: Determine a target movement direction of the two optical display modules.

The target movement direction is a direction in which the two optical display modules move close to or away from each other.

Optionally, the target movement direction may be determined based on the inter pupillary distance adjustment information.

In an implementation, if the inter pupillary distance adjustment information includes the inter pupillary distance offset of the single eye, the target movement direction may be determined based on a positive or negative value of the inter pupillary distance offset. It may be understood that when a current device inter pupillary distance of the head-mounted display device is greater than or less than a user inter pupillary distance, positive and negative values of the inter pupillary distance offset of the single eye may be different. For example, when the device inter pupillary distance is greater than the user inter pupillary distance, the inter pupillary distance offset of the single eye is positive; or when the device inter pupillary distance is less than the user inter pupillary distance, the inter pupillary distance offset of the single eye is negative. Alternatively, on the contrary, when the device inter pupillary distance is greater than the user inter pupillary distance, the inter pupillary distance offset of the single eye is negative; or when the device inter pupillary distance is less than the user inter pupillary distance, the inter pupillary distance offset of the single eye is positive.

In another implementation, if the inter pupillary distance adjustment information includes the target inter pupillary distance value entered by the user, the target movement direction may be determined based on a current inter pupillary distance value of the head-mounted display device and the target inter pupillary distance value entered by the user. When the target inter pupillary distance value is greater than a current inter pupillary distance value, the device inter pupillary distance needs to be increased, and the target movement direction is a direction in which the two optical display modules move away from each other. On the contrary, when the target inter pupillary distance value is less than a current inter pupillary distance value, the target movement direction is a direction in which the two optical display modules move close to each other.

Optionally, if the inter pupillary distance adjustment information includes an inter pupillary distance adjustment direction entered by the user, the inter pupillary distance adjustment direction entered by the user is determined as the target movement direction.

S1103: Control, based on the inter pupillary distance adjustment information and the target movement direction, the IPD adjustment mechanism to operate, to drive the two optical display modules to move.

It can be learned that according to the inter pupillary distance adjustment method provided in this embodiment, the inter pupillary distance adjustment information that indicates an inter pupillary distance adjustment value adjusted by the head-mounted display device may be obtained, and the IPD adjustment mechanism is driven to operate based on the inter pupillary distance adjustment information and the target movement direction, to drive the two optical display modules to move. This implements automatic adjustment on the inter pupillary distance of the head-mounted display device, avoids manual and mechanical adjustment by the user, and improves convenience and accuracy of performing inter pupillary distance adjustment by the head-mounted display device.

Based on the foregoing embodiment, in another embodiment of this application, an implementation of S1103 of controlling the IPD adjustment mechanism to operate based on the inter pupillary distance adjustment information and the target movement direction, to drive the two optical display modules to move is provided.

In an implementation, FIG. 12 is another flowchart of an inter pupillary distance adjustment method according to an embodiment of this application. As shown in FIG. 12, controlling the IPD adjustment mechanism to operate based on the inter pupillary distance adjustment information and the target movement direction, to drive the two optical display modules to move in S1103 may include the following steps.

S1201: Control the IPD adjustment mechanism to operate, to drive the two optical display modules to move in a direction opposite to the target movement direction beyond a preset distance or to move to an inter pupillary distance limit location.

The preset distance is used to eliminate a backlash error introduced by a mechanical structure in the IPD adjustment mechanism. A value of the preset distance is not limited in this embodiment, and may be set based on the backlash error. The preset distance needs to be greater than a straight-line movement distance corresponding to the backlash error.

The inter pupillary distance limit location includes a minimum inter pupillary distance location or a maximum inter pupillary distance location within an adjustable range of the inter pupillary distance of the head-mounted display device.

For the target movement direction, refer to descriptions in S1102. In addition, the target movement direction may alternatively be a preset direction. The preset direction may be a direction in which the two optical display modules move close to or away from each other.

S1202: Control, based on the inter pupillary distance adjustment information, the IPD adjustment mechanism to operate in an opposite direction, to drive the two optical display modules to move in the target movement direction.

An example is used for description with reference to FIG. 2A and FIG. 5A. In FIG. 2A, a current device inter pupillary distance L of the VR glasses 100 is less than a user inter pupillary distance D, and the inter pupillary distance of the VR glasses 100 needs to be increased. In FIG. 5A, when the gear 51 rotates clockwise, the optical display module 1 and the optical display module 2 may be controlled, through the rack 52 and the rack 53, to move away from each other. On the contrary, when the gear 51 rotates counterclockwise, the optical display module 1 and the optical display module 2 move close to each other.

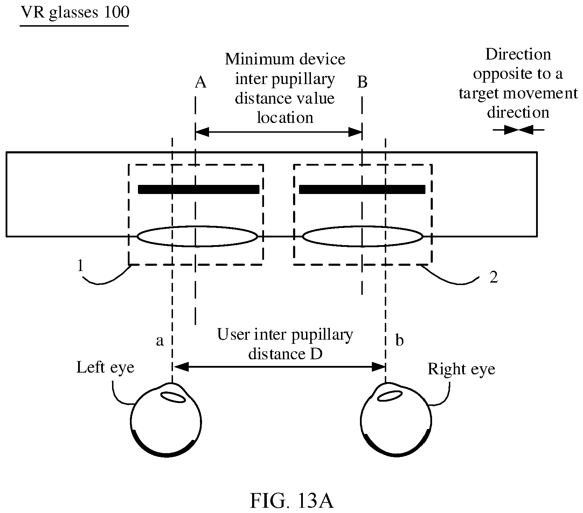

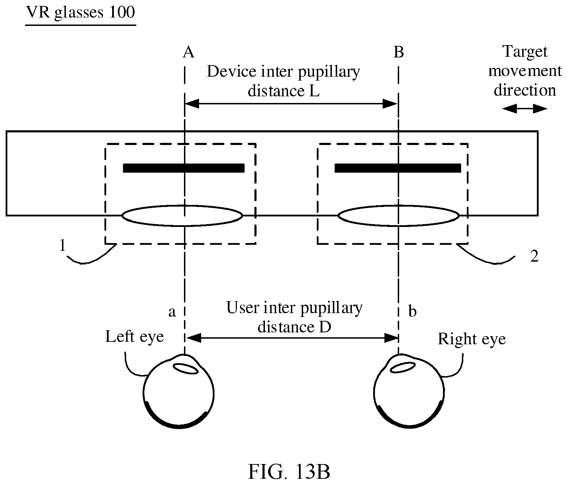

In an example, because the device inter pupillary distance L is less than the user inter pupillary distance D, the inter pupillary distance of the VR glasses 100 needs to be increased, and the target movement direction is determined as a direction in which the optical display module 1 and the optical display module 2 move away from each other. A direction opposite to the target movement direction is a direction in which the optical display module 1 and the optical display module 2 move close to each other. The motor is first controlled to move, and the gear 51 is controlled to rotate counterclockwise, to drive the optical display module 1 and the optical display module 2 to move close to each other, and reach a minimum inter pupillary distance location of the VR glasses 100, as shown in FIG. 13A. Then, the motor is controlled to move in an opposite direction, and the gear 51 is controlled to rotate clockwise, to drive the optical display module 1 and the optical display module 2 to move away from each other. Finally, the device inter pupillary distance L is adjusted to be the same as the user inter pupillary distance D, as shown in FIG. 13B. In a process of adjusting the inter pupillary distance, the user wears the head-mounted display device, and may experience a moving process in which the inter pupillary distance is first decreased and then is increased.

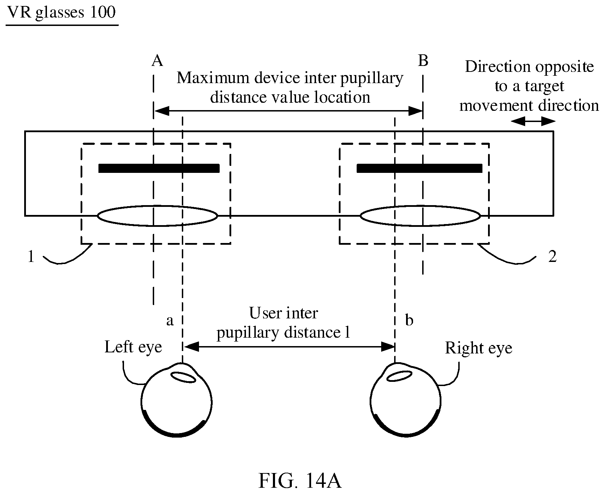

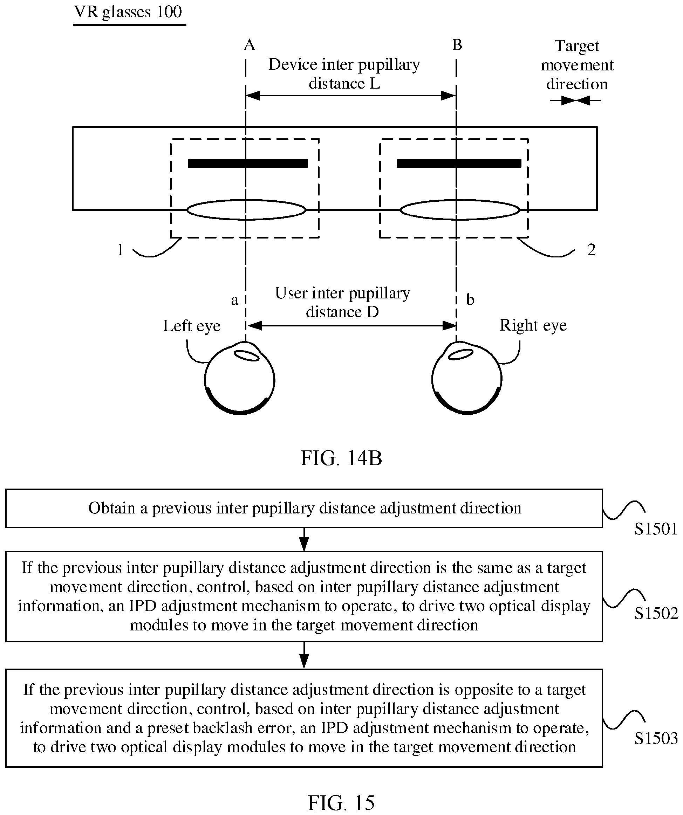

In another example, it is assumed that the target movement direction is a preset direction in which the optical display module 1 and the optical display module 2 move close to each other. A direction opposite to the target movement direction is a direction in which the optical display module 1 and the optical display module 2 move away from each other. The motor is first controlled to move, and the optical display module 1 and the optical display module 2 are controlled to move away from each other, to reach a maximum inter pupillary distance location of the VR glasses 100, as shown in FIG. 14A. Then, the motor is controlled to move in an opposite direction, and the optical display module 1 and the optical display module 2 are controlled to move close to each other. Finally, the device inter pupillary distance L is adjusted to be the same as the user inter pupillary distance D, as shown in FIG. 14B. In a process of adjusting the inter pupillary distance, the user wears the head-mounted display device, and may experience a moving process in which the inter pupillary distance is first increased and then is decreased.

It can be learned that the two optical display modules are first controlled to move by a distance in a direction opposite to the target movement direction, to eliminate influence of a backlash error introduced by a mechanical structure in the IPD adjustment mechanism on inter pupillary distance adjustment of the device, and then, the IPD adjustment mechanism is controlled to operate in an opposite direction, to drive the two optical display modules to move in the target movement direction. This improves precision and accuracy of inter pupillary distance adjustment of the device, and is especially applicable to a scenario in which an accumulated error may be introduced during a plurality of times of forward and reverse adjustment.

In another implementation, FIG. 15 is still another flowchart of an inter pupillary distance adjustment method according to an embodiment of this application. As shown in FIG. 15, controlling the IPD adjustment mechanism to operate based on the inter pupillary distance adjustment information and the target movement direction, to drive the two optical display modules to move in S1103 may include the following steps.

S1501: Obtain a previous inter pupillary distance adjustment direction.

The previous inter pupillary distance adjustment direction is an inter pupillary distance adjustment direction in a latest inter pupillary distance adjustment before a current inter pupillary distance adjustment.

S1502: If the previous inter pupillary distance adjustment direction is the same as the target movement direction, control, based on the inter pupillary distance adjustment information, the IPD adjustment mechanism to operate, to drive the two optical display modules to move in the target movement direction.

S1503: If the previous inter pupillary distance adjustment direction is opposite to the target movement direction, control, based on the inter pupillary distance adjustment information and a preset backlash error, the IPD adjustment mechanism to operate, to drive the two optical display modules to move in the target movement direction.

In this implementation, the target movement direction in a current inter pupillary distance adjustment needs to be compared with an inter pupillary distance adjustment direction in a previous inter pupillary distance adjustment. If movement directions in two consecutive inter pupillary distance adjustments are the same, influence of a backlash error introduced by a mechanical structure in the IPD adjustment mechanism on inter pupillary distance adjustment does not need to be considered, and a current inter pupillary distance adjustment is directly performed based on the target movement direction. If movement directions in two consecutive inter pupillary distance adjustments are opposite, influence of a backlash error introduced by a mechanical structure in the IPD adjustment mechanism on inter pupillary distance adjustment of the device needs to be considered, and a current inter pupillary distance adjustment is still performed based on the target movement direction, but the preset backlash error needs to be considered, to eliminate the backlash error introduced by the mechanical structure in the IPD adjustment mechanism, thereby improving accuracy of inter pupillary distance adjustment of the device.

In still another implementation, controlling, based on the inter pupillary distance adjustment information and the target movement direction, the IPD adjustment mechanism to operate, to drive the two optical display modules to move in S1103 may include the following step:

Control, based on the inter pupillary distance adjustment information, the IPD adjustment mechanism to operate, to drive the two optical display modules to move in the target movement direction.

In this implementation, influence of a backlash error of a mechanical structure in the IPD adjustment mechanism on inter pupillary distance adjustment is not considered, and a current inter pupillary distance adjustment is directly performed based on the target movement direction, thereby implementing automatic adjustment on the device inter pupillary distance.

Optionally, controlling the IPD adjustment mechanism to operate may include:

determining a displacement direction and a displacement distance of a motor in the IPD adjustment mechanism based on the inter pupillary distance adjustment information; and
controlling, based on the displacement direction and the displacement distance of the motor, the motor to move.

Optionally, based on the foregoing embodiment, in still another embodiment of this application, whether the head-mounted display device can end inter pupillary distance adjustment is described.

First, a condition for ending inter pupillary distance adjustment is described.

Optionally, if the head-mounted display device includes an eye tracking apparatus, the condition for ending inter pupillary distance adjustment may include:

An absolute value of a first inter pupillary distance offset is less than or equal to a preset inter pupillary distance offset error. The first inter pupillary distance offset is an inter pupillary distance offset that is of a single eye at a current moment and that is obtained by using the eye tracking apparatus, and is denoted as $\delta x$. The preset inter pupillary distance offset error is denoted as k. The condition may be represented as a formula $|\delta x| \leq k$.

Specifically, a value of the inter pupillary distance offset of the single eye may be a positive value, or may be a negative value. A positive or negative value may reflect whether a current device inter pupillary distance of the head-mounted display device is greater than or less than an inter pupillary distance of human eyes. However, provided that the inter pupillary distance offset of the single eye falls within a first error range, it may be determined that inter pupillary distance adjustment of the device is completed. A value of the preset inter pupillary distance offset error k is not limited in this embodiment. It may be understood that a smaller value of the preset inter pupillary distance offset error k indicates more accurate inter pupillary distance adjustment.

Optionally, if the head-mounted display device includes an IPD adjustment sensor, the condition for ending inter pupillary distance adjustment may include:

An absolute value of a difference between a first detection value and a target detection value is less than or equal to a preset detection value error; and/or an absolute value of a difference between a first inter pupillary distance value and a target inter pupillary distance value is less than or equal to a preset inter pupillary distance error.

The first detection value is a detection value of the IPD adjustment sensor at a current moment, and is denoted as V1. The first inter pupillary distance value is a current inter pupillary distance of the head-mounted display device, and is denoted as L1. The first inter pupillary distance value L1 may be obtained based on the first detection value V1 and the calibration table. The target inter pupillary distance value is an adjusted inter pupillary distance value of the head-mounted display device, and is denoted as L2. The target detection value is a detection value corresponding to the target inter pupillary distance value L2, and is denoted as V2. The target detection value V2 may be obtained based on the target inter pupillary distance value L2 and the calibration table. The preset detection value error is denoted as $\delta V$, and the preset inter pupillary distance error is denoted as $\delta L$. The foregoing two conditions may be respectively represented as a formula $|V2-V1| \leq \delta V$ and a formula $|L2-L1| \leq \delta L$.

Values of the preset detection value error $\delta V$ and the preset inter pupillary distance error $\delta L$ are not

limited in this embodiment. It may be understood that a smaller value indicates more accurate inter pupillary distance adjustment.

Optionally, if different inter pupillary distance adjustment directions respectively correspond to calibration tables, for example, a direction in which the two optical display modules move close to each other corresponds to a first calibration table, and a direction in which the two optical display modules move away from each other corresponds to a second calibration table, when a calibration table is used, calibration tables corresponding to movement directions of the two optical display modules need to be used, to improve accuracy of obtaining a detection value or an inter pupillary distance value. For example, if the target movement direction is a direction in which the two optical display modules move away from each other, the second calibration table is used.

Optionally, the foregoing conditions for ending inter pupillary distance adjustment may be mutually combined. A combination manner is not limited in this embodiment. For example, the head-mounted display device includes an eye tracking apparatus and an IPD adjustment sensor. In some implementations, the condition for ending inter pupillary distance adjustment may include: An absolute value of a first inter pupillary distance offset is less than or equal to a preset inter pupillary distance offset error, and an absolute value of a difference between a first detection value and a target detection value is less than or equal to a preset detection value error. In some other implementations, the condition for ending inter pupillary distance adjustment may include: An absolute value of a first inter pupillary distance offset is less than a preset inter pupillary distance offset error, or an absolute value of a difference between a first inter pupillary distance value and a target inter pupillary distance value is less than a preset inter pupillary distance error.

The following describes an occasion for determining whether the condition for ending inter pupillary distance adjustment is met.

In an implementation, in a process of performing S1103 of controlling, based on the inter pupillary distance adjustment information and the target movement direction, the IPD adjustment mechanism to operate, to drive the two optical display modules to move, it is determined whether the condition for ending inter pupillary distance adjustment is met.

Specifically, whether the condition for ending inter pupillary distance adjustment is met may be periodically determined. A determining period is not limited in this embodiment. If the condition for ending inter pupillary distance adjustment is met, the IPD adjustment mechanism stops operating, movement of the two optical display modules is stopped, and an inter pupillary distance adjustment process is ended. If the condition for ending inter pupillary distance adjustment is not met, S1103 continues to be performed, and whether the condition for ending inter pupillary distance adjustment is met continues to be periodically determined.

In this implementation, excessive movement in one direction in an inter pupillary distance adjustment process is avoided, and a case in which an optical display module needs to be controlled to move in an opposite direction due to excessive movement is avoided.

An example is used for description. It is assumed that the inter pupillary distance of the head-mounted display device needs to be decreased, and the target movement direction is a direction in which the two optical display modules move close to each other. The condition for ending inter pupillary distance adjustment is that an absolute value of a difference between a first detection value V1 and a target detection value V2 is less than a preset detection value error $\delta V$. In a process of controlling the two optical display modules to move close to each other, a first detection value V11 is obtained at a moment 11, and whether |V11-V2| is less than $\delta V$ is determined. If |V11-V2| is not less than $\delta V$, the two optical display modules continue to be controlled to move close to each other. At a moment t1+T, a first detection value V12 is obtained, and whether |V12-V21 is less than $\delta V$ is determined. If |V12-V2| is not less than $\delta V$, the two optical display modules continue to be controlled to move close to each other. At a moment t1+2*T, a first detection value V13 is obtained, and whether |V13-V2| is less than $\delta V$ is determined. If |V13-V2| is less than $\delta V$, the IPD adjustment mechanism is controlled to stop operating, and movement of the two optical display modules is controlled to stop.

In another implementation, after S1103, the method may further include: determining whether the condition for ending inter pupillary distance adjustment is met. If the condition for ending inter pupillary distance adjustment is met, a procedure ends, and automatic adjustment on the inter pupillary distance of the head-mounted display device is completed. If the condition for ending inter pupillary distance adjustment is not met, return to S1103 and perform S1103.

Specifically, the displacement direction and the displacement distance of the motor in the IPD adjustment mechanism or a moving distance of the two optical display modules may be determined based on the inter pupillary distance adjustment information. S1103 is performed to control the motor to move to a target location corresponding to the inter pupillary distance adjustment information or to control the two optical display modules to move to target locations corresponding to the inter pupillary distance adjustment information. It may be understood that in this case, a difference between the device inter pupillary distance of the head-mounted display device and the user inter pupillary distance may be small, and the condition for ending inter pupillary distance adjustment is met; or a difference between the device inter pupillary distance of the head-mounted display device and the user inter pupillary distance may be large, and the condition for ending inter pupillary distance adjustment is not met. When the condition for ending inter pupillary distance adjustment is not met, the two optical display modules may move by an excessive distance or move by an inadequate distance in the target movement direction.

An example is used for description. It is assumed that the inter pupillary distance of the head-mounted display device needs to be decreased, and the target movement direction is a direction in which the two optical display modules move close to each other. The displacement direction and the displacement distance of the motor in the IPD adjustment mechanism are determined based on the inter pupillary distance adjustment information. When the motor moves in the displacement direction to reach the displacement distance, the two optical display modules are driven to move close to each other by a distance. In this case, the two optical display modules may move by an inadequate distance in a direction in which the two optical display modules move close to each other. As a result, the inter pupillary distance of the head-mounted display device is still much greater than the user inter pupillary distance, and under-movement occurs. Alternatively, the two optical display modules move by an excessively large distance in a direction in which the two optical display modules move close to each other. As a result, the inter pupillary distance of the head-mounted display device is already less than the user inter pupillary distance and is much less than the user inter pupillary distance. In this case, the two optical display modules need to move in an opposite direction by a distance, to increase the device inter pupillary distance, and excessive movement occurs.

In this implementation, after S1103 is performed, whether the condition for ending inter pupillary distance adjustment is met is determined. If the condition is met, a procedure ends. If the condition is not met, return to S1103 and perform S1103, and whether the condition for ending inter pupillary distance adjustment is met continues to be performed until the condition for ending inter pupillary distance adjustment is met. When excessive movement occurs, and the device inter pupillary distance needs to be adjusted in an opposite movement direction next time, refer to the method provided in FIG. 15. When movement directions in two adjacent times of inter pupillary distance adjustment are opposite, influence of a backlash error introduced by a mechanical structure in the IPD adjustment mechanism on inter pupillary distance adjustment of the device is considered.

Optionally, the inter pupillary distance adjustment method provided in this embodiment may further include:

outputting prompt information, where the prompt information indicates that inter pupillary distance adjustment ends.

A manner of outputting the prompt information is not limited in this embodiment. For example, the information may be output through voice, or may be displayed in a related interface. Refer to FIG. 10C or FIG. 10D.

Optionally, the prompt information may include an adjusted device inter pupillary distance value.

Optionally, based on the foregoing embodiment, when the head-mounted display device includes different hardware structures, an implementation procedure of the inter pupillary distance adjustment method is provided.

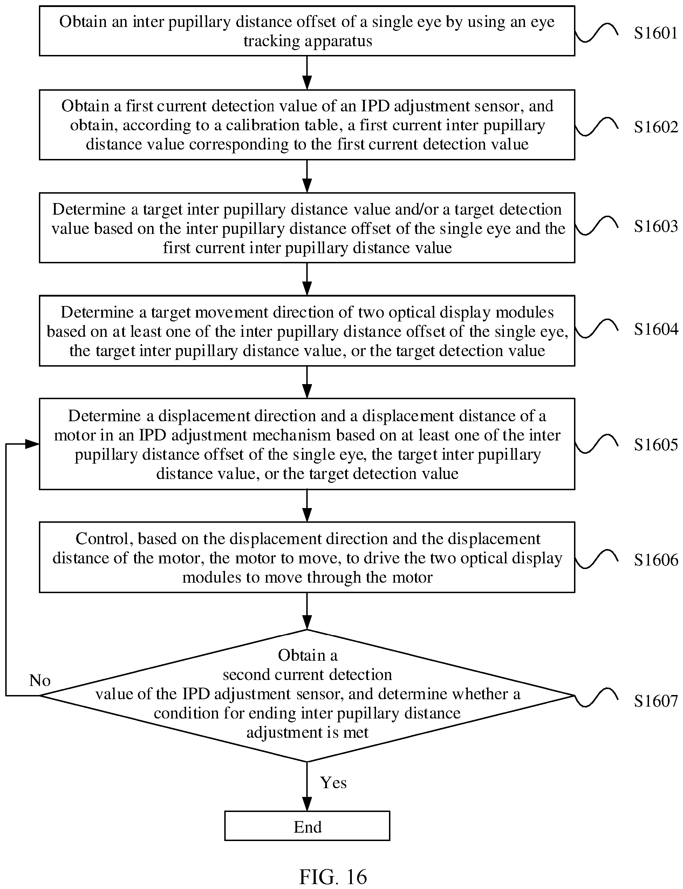

FIG. 16 is still another flowchart of an inter pupillary distance adjustment method according to an embodiment of this application. In this implementation, a head-mounted display device includes two optical display modules, an IPD adjustment mechanism, an eye tracking apparatus, and an IPD adjustment sensor. As shown in FIG. 16, the inter pupillary distance adjustment method provided in this embodiment may include the following steps.

S1601: Obtain an inter pupillary distance offset of a single eye by using the eye tracking apparatus. The inter pupillary distance offset of the single eye indicates a deviation between a pupil of the single eye and a corresponding optical display module.

S1602: Obtain a first current detection value of the IPD adjustment sensor, and obtain, according to a calibration table, a first current inter pupillary distance value corresponding to the first current detection value. The calibration table indicates a correspondence between a detection value of the IPD adjustment sensor and an inter pupillary distance of the head-mounted display device.

An execution sequence of S1601 and S1602 is not limited.

S1603: Determine a target inter pupillary distance value and/or a target detection value based on the inter pupillary distance offset of the single eye and the first current inter pupillary distance value.

Specifically, the target inter pupillary distance value may be determined based on the first current inter pupillary distance value and the inter pupillary distance offset of the single eye. The target detection value corresponding to the target inter pupillary distance value may be obtained according to the calibration table.

S1604: Determine a target movement direction of the two optical display modules based on at least one of the inter pupillary distance offset of the single eye, the target inter pupillary distance value, or the target detection value.

S1605: Determine a displacement direction and a displacement distance of a motor in the IPD adjustment mechanism based on at least one of the inter pupillary distance offset of the single eye, the target inter pupillary distance value, or the target detection value.

S1606: Control, based on the displacement direction and the displacement distance of the motor, the motor to move, to drive the two optical display modules to move through the motor.

S1607: Obtain a second current detection value of the IPD adjustment sensor, and determine whether a condition for ending inter pupillary distance adjustment is met.

Optionally, in an implementation, whether an absolute value of a difference between the second current detection value and the target detection value is less than a preset detection value error is determined. If the absolute value is less than the preset detection value error, it is determined that the condition for ending inter pupillary distance adjustment is met, and a procedure stops. If the absolute value is greater than or equal to the preset detection value error, it is determined that the condition for ending inter pupillary distance adjustment is not met. In this case, return to S1605 and perform S1605.

Optionally, in another implementation, a second current inter pupillary distance value corresponding to the second current detection value is obtained according to the calibration table, and whether an absolute value of a difference between the second current inter pupillary distance value and the target inter pupillary distance value is less than a preset inter pupillary distance error needs to be determined. If the absolute value is less than the preset inter pupillary distance error, it is determined that the condition for ending inter pupillary distance adjustment is met, and a procedure stops. If the absolute value is greater than or equal to the preset inter pupillary distance error, it is determined that the condition for ending inter pupillary distance adjustment is not met. In this case, return to S1605 and perform S1605.

It can be learned that, in this implementation, the inter pupillary distance offset of the single eye is obtained by using the eye tracking apparatus, and the target inter pupillary distance value and/or the target detection value are/is determined based on the inter pupillary distance offset of the single eye and the calibration table, thereby improving accuracy of obtaining an inter pupillary distance adjustment value of the device. In addition, the IPD adjustment mechanism is controlled to operate based on the value, thereby improving accuracy of automatic adjustment on the inter pupillary distance of the device. In addition, it is determined, based on the detection value of the IPD adjustment sensor and the calibration table, whether inter pupillary distance adjustment of the device can be ended, thereby further improving accuracy of performing inter pupillary distance adjustment by the head-mounted display device.

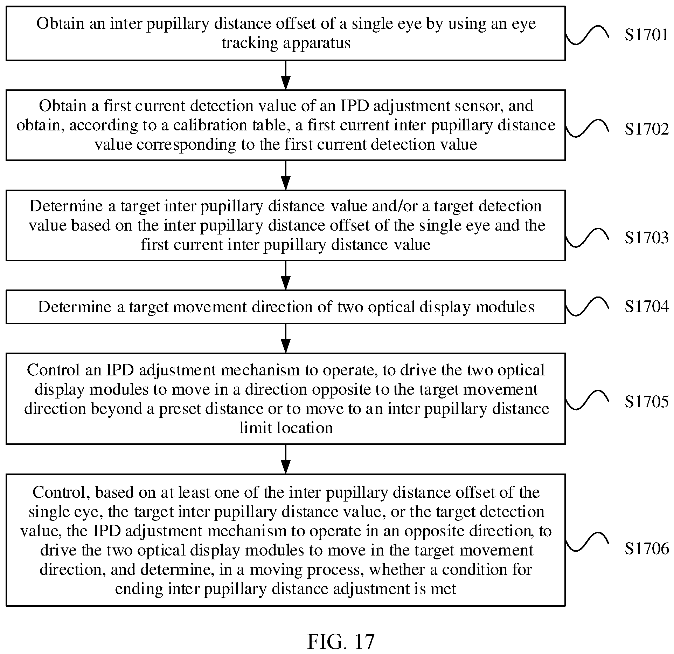

FIG. 17 is still another flowchart of an inter pupillary distance adjustment method according to an embodiment of this application. In this implementation, a head-mounted display device includes two optical display modules, an IPD adjustment mechanism, an eye tracking apparatus, and an IPD adjustment sensor. As shown in FIG. 17, the inter pupillary distance adjustment method provided in this embodiment may include the following steps.

S1701: Obtain an inter pupillary distance offset of a single eye by using the eye tracking apparatus. The inter pupillary distance offset of the single eye indicates a deviation between a pupil of the single eye and a corresponding optical display module.

S1702: Obtain a first current detection value of the IPD adjustment sensor, and obtain, according to a calibration table, a first current inter pupillary distance value corresponding to the first current detection value. The calibration table indicates a correspondence between a detection value of the IPD adjustment sensor and an inter pupillary distance of the head-mounted display device.

An execution sequence of S1701 and S1702 is not limited.

S1703: Determine a target inter pupillary distance value and/or a target detection value based on the inter pupillary distance offset of the single eye and the first current inter pupillary distance value.

Specifically, the target inter pupillary distance value may be determined based on the first current inter pupillary distance value and the inter pupillary distance offset of the single eye. The target detection value corresponding to the target inter pupillary distance value may be obtained according to the calibration table.

S1704: Determine a target movement direction of the two optical display modules.

Optionally, the target movement direction of the two optical display modules may be determined based on at least one of the inter pupillary distance offset of the single eye, the target inter pupillary distance value, or the target detection value.

Optionally, the target movement direction may be a preset direction. The preset direction is a direction in which the two optical display modules move close to or away from each other.

S1705: Control the IPD adjustment mechanism to operate, to drive the two optical display modules to move in a direction opposite to the target movement direction beyond a preset distance or to move to an inter pupillary distance limit location.

S1706: Control, based on at least one of the inter pupillary distance offset of the single eye, the target inter pupillary distance value, or the target detection value, the IPD adjustment mechanism to operate in an opposite direction, to drive the two optical display modules to move in the target movement direction, and determine, in a moving process, whether a condition for ending inter pupillary distance adjustment is met.

For determining whether the condition for ending inter pupillary distance adjustment is met, refer to related descriptions of S1607. Details are not described herein again.

If the condition for ending inter pupillary distance adjustment is met, a procedure stops. If the condition for ending inter pupillary distance adjustment is not met, S1706 continues to be executed.

It can be learned that, in this implementation, the two optical display modules are first controlled to move by a distance in a direction opposite to the target movement direction, to eliminate influence of a backlash error introduced by a mechanical structure in the IPD adjustment mechanism on inter pupillary distance adjustment of the device, and then, the IPD adjustment mechanism is controlled to operate in an opposite direction, to drive the two optical display modules to move in the target movement direction, thereby improving precision and accuracy of inter pupillary distance adjustment of the device.

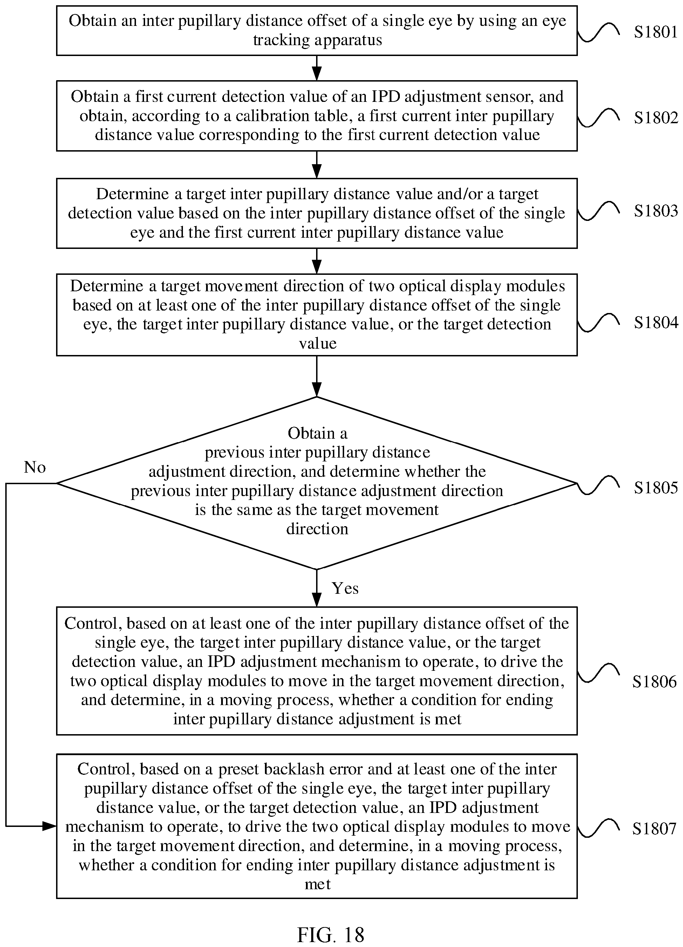

FIG. 18 is still another flowchart of an inter pupillary distance adjustment method according to an embodiment of this application. In this implementation, a head-mounted display device includes two optical display modules, an IPD adjustment mechanism, an eye tracking apparatus, and an IPD adjustment sensor. As shown in FIG. 18, the inter pupillary distance adjustment method provided in this embodiment may include the following steps.

S1801: Obtain an inter pupillary distance offset of a single eye by using the eye tracking apparatus. The inter pupillary distance offset of the single eye indicates a deviation between a pupil of the single eye and a corresponding optical display module.

S1802: Obtain a first current detection value of the IPD adjustment sensor, and obtain, according to a calibration table, a first current inter pupillary distance value corresponding to the first current detection value. The calibration table indicates a correspondence between a detection value of the IPD adjustment sensor and an inter pupillary distance of the head-mounted display device.

An execution sequence of S1801 and S1802 is not limited.

S1803: Determine a target inter pupillary distance value and/or a target detection value based on the inter pupillary distance offset of the single eye and the first current inter pupillary distance value.

Specifically, the target inter pupillary distance value may be determined based on the first current inter pupillary distance value and the inter pupillary distance offset of the single eye. The target detection value corresponding to the target inter pupillary distance value may be obtained according to the calibration table.

S1804: Determine a target movement direction of the two optical display modules based on at least one of the inter pupillary distance offset of the single eye, the target inter pupillary distance value, or the target detection value.

S1805: Obtain a previous inter pupillary distance adjustment direction, and determine whether the previous inter pupillary distance adjustment direction is the same as the target movement direction.

If the previous inter pupillary distance adjustment direction is the same as the target movement direction, S1806 is performed. Otherwise, if the previous inter pupillary distance adjustment direction is different from the target movement direction, S1807 is performed.

S1806: If the previous inter pupillary distance adjustment direction is the same as the target movement direction, control, based on at least one of the inter pupillary distance offset of the single eye, the target inter pupillary distance value, or the target detection value, the IPD adjustment mechanism to operate, to drive the two optical display modules to move in the target movement direction, and determine, in a moving process, whether a condition for ending inter pupillary distance adjustment is met.

If the condition for ending inter pupillary distance adjustment is met, a procedure stops. If the condition for ending inter pupillary distance adjustment is not met, S1806 continues to be executed.

S1807: If the previous inter pupillary distance adjustment direction is opposite to the target movement direction, control, based on a preset backlash error and at least one of the inter pupillary distance offset of the single eye, the target inter pupillary distance value, or the target detection value, the IPD adjustment mechanism to operate, to drive the two optical display modules move in the target movement direction, and determine, in a moving process, whether a condition for ending inter pupillary distance adjustment is met.

If the condition for ending inter pupillary distance adjustment is met, a procedure stops. If the condition for ending inter pupillary distance adjustment is not met, S1807 continues to be executed.

For determining whether the condition for ending inter pupillary distance adjustment is met, refer to related descriptions of S1607. Details are not described herein again.

It can be learned that, in this implementation, the target movement direction in a current inter pupillary distance adjustment needs to be compared with an inter pupillary distance adjustment direction in a previous inter pupillary distance adjustment. If directions in two consecutive inter pupillary distance adjustments are the same, influence of a backlash error introduced by a mechanical structure in the IPD adjustment mechanism on inter pupillary distance adjustment does not need to be considered, and a current inter pupillary distance adjustment is directly performed based on the target movement direction. If directions in two consecutive inter pupillary distance adjustments are opposite, influence of a backlash error introduced by a mechanical structure in the IPD adjustment mechanism on inter pupillary distance adjustment of the device needs to be considered, and a current inter pupillary distance adjustment is still performed based on the target movement direction, but the preset backlash error needs to be considered, to eliminate the backlash error introduced by the mechanical structure in the IPD adjustment mechanism, thereby improving accuracy of inter pupillary distance adjustment of the device.

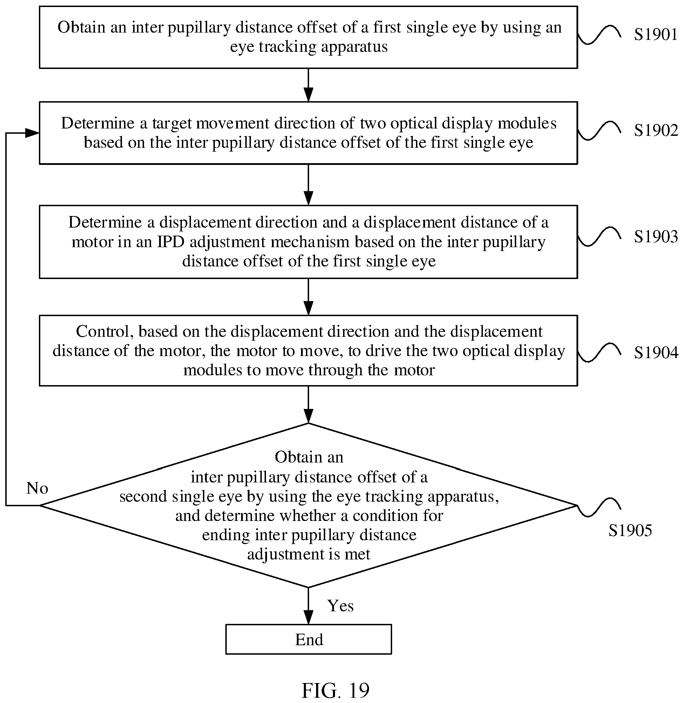

FIG. 19 is still another flowchart of an inter pupillary distance adjustment method according to an embodiment of this application. In this implementation, a head-mounted display device includes two optical display modules, an IPD adjustment mechanism, and an eye tracking apparatus. As shown in FIG. 19, the inter pupillary distance adjustment method provided in this embodiment may include the following steps.

S1901: Obtain an inter pupillary distance offset of a first single eye by using the eye tracking apparatus. An inter pupillary distance offset of a single eye indicates a deviation between a pupil of the single eye and a corresponding optical display module.

S1902: Determine a target movement direction of the two optical display modules based on the inter pupillary distance offset of the first single eye.

S1903: Determine a displacement direction and a displacement distance of a motor in the IPD adjustment mechanism based on the inter pupillary distance offset of the first single eye.

S1904: Control the motor to move based on the displacement direction and the displacement distance of the motor, to drive the two optical display modules to move through the motor.

S1905: Obtain an inter pupillary distance offset of a second single eye by using the eye tracking apparatus, and determine whether a condition for ending inter pupillary distance adjustment is met.

Specifically, whether an absolute value of the inter pupillary distance offset of the second single eye is less than a preset inter pupillary distance offset error is determined. If the absolute value is less than the preset inter pupillary distance offset error, it is determined that the condition for ending inter pupillary distance adjustment is met, and a procedure stops. If the absolute value is greater than or equal to the preset inter pupillary distance offset error, it is determined that the condition for ending inter pupillary distance adjustment is not met. In this case, return to S1902 and perform S1902, and the inter pupillary distance offset of the second single eye is the inter pupillary distance offset of the first single eye in S1902.

It can be learned that, in this implementation, the head-mounted display device includes the eye tracking apparatus, the inter pupillary distance offset of the single eye is obtained by using the eye tracking apparatus, an inter pupillary distance adjustment value adjusted by the head-mounted display device is determined based on the inter pupillary distance offset of the single eye, and whether inter pupillary distance adjustment of the device can be ended is determined, thereby improving accuracy of performing inter pupillary distance adjustment by the head-mounted display device.

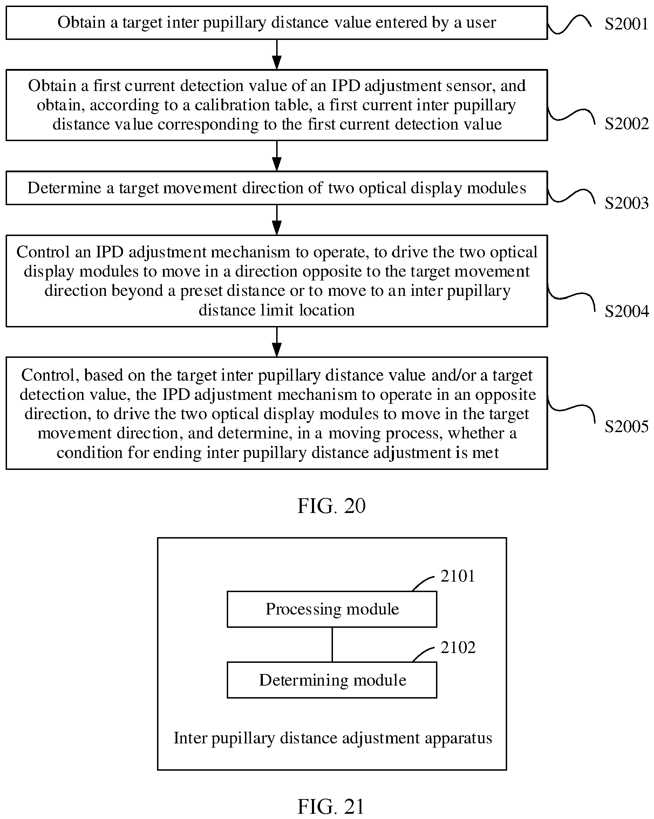

FIG. 20 is still another flowchart of an inter pupillary distance adjustment method according to an embodiment of this application. In this implementation, a head-mounted display device includes two optical display modules, an IPD adjustment mechanism, and an IPD adjustment sensor. As shown in FIG. 20, the inter pupillary distance adjustment method provided in this embodiment may include the following steps.

S2001: Obtain a target inter pupillary distance value entered by a user.

S2002: Obtain a first current detection value of the IPD adjustment sensor, and obtain, according to a calibration table, a first current inter pupillary distance value corresponding to the first current detection value. The calibration table indicates a correspondence between a detection value of the IPD adjustment sensor and an inter pupillary distance of the head-mounted display device.

An execution sequence of S2001 and S2002 is not limited.

S2003: Determine a target movement direction of the two optical display modules.

Optionally, the target movement direction of the two optical display modules may be determined based on the first current inter pupillary distance value and the target inter pupillary distance value.

Optionally, the target movement direction may be a preset direction. The preset direction is a direction in which the two optical display modules move close to or away from each other.

S2004: Control the IPD adjustment mechanism to operate, to drive the two optical display modules to move in a direction opposite to the target movement direction beyond a preset distance or to move to an inter pupillary distance limit location.

S2005: Control, based on the target inter pupillary distance value and/or a target detection value, the IPD adjustment mechanism to operate in an opposite direction, to drive the two optical display modules to move in the target movement direction, and determine, in a moving process, whether a condition for ending inter pupillary distance adjustment is met.

The target detection value corresponding to the target inter pupillary distance value may be obtained according to the calibration table.

For determining whether the condition for ending inter pupillary distance adjustment is met, refer to related descriptions of S1607. Details are not described herein again.

If the condition for ending inter pupillary distance adjustment is met, a procedure stops. If the condition for ending inter pupillary distance adjustment is not met, S2005 continues to be executed.

It can be learned that, in this implementation, the user enters the target inter pupillary distance value of the head-mounted display device. When a device inter pupillary distance is adjusted, the two optical display modules are first controlled to move by a distance in a direction opposite to the target movement direction, to eliminate influence of a backlash error introduced by a mechanical structure in the IPD adjustment mechanism on inter pupillary distance adjustment of the device, and then, the IPD adjustment mechanism is controlled to operate in an opposite direction, to drive the two optical display modules to move in the target movement direction, thereby improving precision and accuracy of inter pupillary distance adjustment of the device.

It may be understood that, to implement the foregoing functions, the head-mounted display device includes corresponding hardware and/or software modules for performing the functions. With reference to algorithm steps of examples described in embodiments disclosed in this specification, this application can be implemented in a form of hardware or a combination of hardware and computer software. Whether a function is performed by hardware or hardware driven by computer software depends on particular applications and design constraints of the technical solutions. A person skilled in the art may use different methods to implement the described functions for each particular application with reference to embodiments, but it should not be considered that the implementation goes beyond the scope of this application.

In embodiments of this application, the head-mounted display device may be divided into functional modules based on the foregoing method examples. For example, each functional module may be obtained through division based on each corresponding function, or two or more functions may be integrated into one processing module. It should be noted that, in embodiments of this application, division into modules is an example, and is merely a logical function division. In actual implementation, another division manner may be used. It should be noted that a name of a module in embodiments of this application is an example, and the name of the module is not limited during actual implementation.

For example, FIG. 21 is a diagram of a structure of an inter pupillary distance adjustment apparatus according to an embodiment of this application. The inter pupillary distance adjustment apparatus provided in this embodiment may be used in a head-mounted display device. The head-mounted display device includes an IPD adjustment mechanism and two optical display modules, and the IPD adjustment mechanism is configured to drive the two optical display modules to move. As shown in FIG. 21, the inter pupillary distance adjustment apparatus provided in this embodiment may include:

a processing module 2101, configured to: after a user wears the head-mounted display device, control the IPD adjustment mechanism to move by a first distance in a first direction, where the first distance is used to eliminate a backlash error of the IPD adjustment mechanism.

The processing module 2101 is further configured to: control the IPD adjustment mechanism to operate and move by a second distance in a second direction, to drive the two optical display modules to move until a determining module 2102 determines that a deviation between an inter pupillary distance of the head-mounted display device and an inter pupillary distance of the user falls within a first error range.

In a possible implementation, the first direction is opposite to the second direction, and the first direction is a direction in which the two optical display modules move close to or away from each other.

In a possible implementation, the first direction is a preset direction, and the preset direction is the direction in which the two optical display modules move close to or away from each other.

In a possible implementation, the processing module 2101 is further configured to:
control the IPD adjustment mechanism to operate and move by the first distance in the first direction, to drive the two optical display modules to move beyond a preset distance or to move to an inter pupillary distance limit location.

In a possible implementation, the first direction is the same as the second direction, and the first direction is a direction in which the two optical display modules move close to or away from each other.

In a possible implementation, the first direction and the second direction are opposite to an operation direction of the IPD adjustment mechanism during a previous inter pupillary distance adjustment of the head-mounted display device, and the first distance is 0; and the processing module 2101 is configured to:
control the IPD adjustment mechanism to operate for first duration in the first direction, where the first duration is used to eliminate the backlash error of the IPD adjustment mechanism.

In a possible implementation, the IPD adjustment mechanism includes a motor, a gear connected to the motor, and a rack connected to the gear, and the rack is further connected to the optical display module. The processing module 2101 is configured to:
control the motor to operate for the first duration in the first direction, to drive the gear to rotate for the first duration.

In a possible implementation, the first direction and the second direction are the same as an operation direction of the IPD adjustment mechanism during a previous inter pupillary distance adjustment of the head-mounted display device, and the first distance is 0; and the processing module 2101 is configured to:
control the IPD adjustment mechanism not to operate.

In a possible implementation, the processing module 2101 is further configured to:

obtain inter pupillary distance adjustment information before controlling the IPD adjustment mechanism to move by the first distance in the first direction, where the inter pupillary distance adjustment information represents an inter pupillary distance adjustment value adjusted by the head-mounted display device; and
determine the first direction and/or the second direction based on the inter pupillary distance adjustment information.

In a possible implementation, the head-mounted display device further includes an eye tracking apparatus, and the processing module 2101 is configured to:
obtain an inter pupillary distance offset of a single eye by using the eye tracking apparatus, where the inter pupillary distance offset of the single eye indicates a deviation between a pupil of the single eye and a corresponding optical display module.

In a possible implementation, the processing module 2101 is further configured to:

perform eye tracking on a left eye or a right eye of a person by using the eye tracking apparatus, and determine an obtained inter pupillary distance offset of the left eye or an obtained inter pupillary distance offset of the right eye as the inter pupillary distance offset of the single eye.

In a possible implementation, the processing module 2101 is further configured to:

separately perform eye tracking on a left eye and a right eye of a person by using the eye tracking apparatus, and determine the inter pupillary distance offset of the single eye based on an obtained inter pupillary distance offset of the left eye and an obtained inter pupillary distance offset of the right eye.

In a possible implementation, the processing module 2101 is further configured to:

determine the inter pupillary distance offset of the left eye and the inter pupillary distance offset of the right eye as the inter pupillary distance offset of the single eye; or determine an average value of the inter pupillary distance offset of the left eye and the inter pupillary distance offset of the right eye as the inter pupillary distance offset of the single eye.

In a possible implementation, the head-mounted display device further includes an IPD adjustment sensor, and a detection value of the IPD adjustment sensor represents the inter pupillary distance of the head-mounted display device; and the processing module 2101 is further configured to:

obtain a current detection value of the IPD adjustment sensor; and
determine a target inter pupillary distance value and/or a target detection value based on a calibration table, the inter pupillary distance offset of the single eye, and the current detection value, where the calibration table indicates a correspondence between the detection value of the IPD adjustment sensor and the inter pupillary distance of the head-mounted display device.

In a possible implementation, the processing module 2101 is further configured to:

obtain a target inter pupillary distance value entered by the user.

In a possible implementation, the processing module 2101 is further configured to:

obtain a target inter pupillary distance value entered by the user through voice.

In a possible implementation, the processing module 2101 is further configured to:

display a first interface; and
obtain a target inter pupillary distance value entered by the user in the first interface.

In a possible implementation, the head-mounted display device further includes the eye tracking apparatus, and that the deviation between the inter pupillary distance of the head-mounted display device and the inter pupillary distance of the user falls within the first error range includes:

$|\delta x| \leq k$, where $\delta x$ represents an inter pupillary distance offset that is of the single eye at a current moment and that is obtained by using the eye tracking apparatus, and $k$ represents a preset inter pupillary distance offset error.

In a possible implementation, the head-mounted display device further includes the IPD adjustment sensor, and the detection value of the IPD adjustment sensor represents the inter pupillary distance of the head-mounted display device; and that the deviation between the inter pupillary distance of the head-mounted display device and the inter pupillary distance of the user falls within the first error range includes:

$|V2-V1| \leq \delta V$, and/or $|L2-L1| \leq \delta L$, where V1 represents a first detection value of the IPD adjustment sensor at a current moment, V2 represents the target detection value corresponding to a target inter pupillary distance of the head-mounted display device, L1 represents a first inter pupillary distance value that corresponds to the first detection value V1 and that is determined according to the calibration table, L2 represents the target inter pupillary distance value, $\delta V$ represents a preset detection value error, $\delta L$ represents a preset inter pupillary distance error, and the calibration table indicates the correspondence between the detection value of the IPD adjustment sensor and the inter pupillary distance of the head-mounted display device.

In a possible implementation, the calibration table includes a first calibration table and a second calibration table, the first calibration table corresponds to a movement direction in which the two optical display modules move close to each other, and the second calibration table corresponds to a movement direction in which the two optical display modules move away from each other.

In a possible implementation, the processing module 2101 is further configured to:

output prompt information, where the prompt information indicates that inter pupillary distance adjustment of the head-mounted display device ends.

In a possible implementation, the prompt information further includes an inter pupillary distance value of the head-mounted display device after the inter pupillary distance adjustment.

Technical principles and technical effects of the inter pupillary distance adjustment method provided in this embodiment are similar to those of inter pupillary distance adjustment methods provided in method embodiments of this application. Details are not described herein again.

An embodiment of this application provides a computer program product. When the computer program product runs on a device, the device is enabled to perform technical solutions in the foregoing embodiments. Implementation principles and technical effects thereof are similar to those of the foregoing related embodiments. Details are not described herein again.

An embodiment of this application provides a computer-readable storage medium. The computer-readable storage medium stores program instructions. When the program instructions are executed by a device, the device is enabled to perform technical solutions in the foregoing embodiments. Implementation principles and technical effects thereof are similar to those of the foregoing related embodiments. Details are not described herein again.

In conclusion, the foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application.

## Claims

1. An inter pupillary distance adjustment method, applied to a head-mounted display device, wherein the head-mounted display device comprises an inter pupillary distance IPD adjustment mechanism and two optical display modules, and the IPD adjustment mechanism is configured to drive the two optical display modules to move; and the method comprises:

   after a user wears the head-mounted display device, controlling the IPD adjustment mechanism to move by a first distance in a first direction, wherein the first distance is used to eliminate a backlash error of the IPD adjustment mechanism; and
   controlling the IPD adjustment mechanism to operate and move by a second distance in a second direction, wherein the IPD adjustment mechanism drives the two optical display modules to move, and a distance between the two optical display modules after the movement matches an inter pupillary distance of the user.

2. The method according to claim 1, wherein the first direction is opposite to the second direction, and the first direction is a direction in which the two optical display modules move close to or away from each other.

3. The method according to claim 2, wherein the first direction is a preset direction, and the preset direction is the direction in which the two optical display modules move close to or away from each other.

4. The method according to claim 2, wherein the controlling the IPD adjustment mechanism to move by a first distance in a first direction comprises:
   controlling the IPD adjustment mechanism to operate and move by the first distance in the first direction, to drive the two optical display modules to move beyond a preset distance or to move to an inter pupillary distance limit location.

5. The method according to claim 1, wherein the first direction is the same as the second direction, and the first direction is a direction in which the two optical display modules move close to or away from each other.

6. The method according to claim 5, wherein the first direction and the second direction are opposite to an operation direction of the IPD adjustment mechanism during a previous inter pupillary distance adjustment of the head-mounted display device, and the first distance is 0; and the controlling the IPD adjustment mechanism to move by a first distance in a first direction comprises:
   controlling the IPD adjustment mechanism to operate for first duration in the first direction, wherein the first duration is used to eliminate the backlash error of the IPD adjustment mechanism.

7. The method according to claim 6, wherein the IPD adjustment mechanism comprises a motor, a gear connected to the motor, and a rack connected to the gear, and the rack is further connected to the optical display module; and the controlling the IPD adjustment mechanism operate for first duration in the first direction comprises:
   controlling the motor to operate for the first duration in the first direction, to drive the gear to rotate for the first duration.

8. The method according to any one of claims 1 and 2 and 4 to 7, wherein before the controlling the IPD adjustment mechanism to move by a first distance in a first direction, the method further comprises:

   obtaining inter pupillary distance adjustment information, wherein the inter pupillary distance adjustment information represents an inter pupillary distance adjustment value adjusted by the head-mounted display device; and
   determining the first direction and/or the second direction based on the inter pupillary distance adjustment information.

9. The method according to claim 8, wherein the head-mounted display device further comprises an eye tracking apparatus, and the obtaining inter pupillary distance adjustment information comprises:
   obtaining an inter pupillary distance offset of a single eye by using the eye tracking apparatus, wherein the inter pupillary distance offset of the single eye indicates a deviation between a pupil of the single eye and a corresponding optical display module.

10. The method according to claim 9, wherein the head-mounted display device further comprises an IPD adjustment sensor, and a detection value of the IPD adjustment sensor represents an inter pupillary distance of the head-mounted display device; and the obtaining inter pupillary distance adjustment information further comprises:

obtaining a current detection value of the IPD adjustment sensor; and
determining a target inter pupillary distance value and/or a target detection value based on a calibration table, the inter pupillary distance offset of the single eye, and the current detection value, wherein the calibration table indicates a correspondence between the detection value of the IPD adjustment sensor and the inter pupillary distance of the head-mounted display device.

11. The method according to any one of claims 1 to 10, wherein the head-mounted display device further comprises the eye tracking apparatus, and that the distance between the two optical display modules matches the inter pupillary distance of the user comprises:
$|\delta x| \leq k$, wherein $\delta x$ represents an inter pupillary distance offset that is of the single eye at a current moment and that is obtained by using the eye tracking apparatus, and k represents a preset inter pupillary distance offset error.

12. The method according to any one of claims 1 to 10, wherein the head-mounted display device further comprises the IPD adjustment sensor, and the detection value of the IPD adjustment sensor represents the inter pupillary distance of the head-mounted display device; and that the distance between the two optical display modules matches the inter pupillary distance of the user comprises:

$$|V2{-}V1| \leq \delta V, \text{ and/or } |L2{-}L1| \leq \delta L,$$

wherein
V1 represents a first detection value of the IPD adjustment sensor at a current moment, V2 represents the target detection value corresponding to a target inter pupillary distance of the head-mounted display device, L1 represents a first inter pupillary distance value that corresponds to the first detection value V1 and that is determined according to the calibration table, L2 represents the target inter pupillary distance value, $\delta V$ represents a preset detection value

error, $\delta L$ represents a preset inter pupillary distance error, and the calibration table indicates the correspondence between the detection value of the IPD adjustment sensor and the inter pupillary distance of the head-mounted display device.

13. The method according to any one of claims 1 to 12, wherein the method further comprises:
outputting prompt information, wherein the prompt information indicates that inter pupillary distance adjustment of the head-mounted display device ends.

14. The method according to claim 13, wherein the prompt information further comprises an inter pupillary distance value of the head-mounted display device after the inter pupillary distance adjustment.

15. A head-mounted display device, comprising: an inter pupillary distance IPD adjustment mechanism, two optical display modules, a processor, and a memory, wherein

the IPD adjustment mechanism is configured to drive the two optical display modules to move; and
the processor is configured to: be coupled to the memory, read instructions in the memory, and enable, according to the instructions, the head-mounted display device to perform the method according to any one of claims 1 to 14.

16. The head-mounted display device according to claim 15, wherein the head-mounted display device further comprises an eye tracking apparatus, and the eye tracking apparatus comprises at least one light source and at least one camera module;

the at least one light source is configured to emit light to a human eye; and
the at least one camera module is configured to obtain a part of the light reflected after the at least one light source irradiates the eye.

17. The head-mounted display device according to claim 15 or 16, wherein the head-mounted display device further comprises an IPD adjustment sensor; and
a detection value of the IPD adjustment sensor represents an inter pupillary distance of the head-mounted display device.

18. The head-mounted display device according to any one of claims 15 to 17, wherein the IPD adjustment mechanism comprises a motor and an IPD adjustment mechanical structure connected to the motor, the IPD adjustment mechanical structure comprises a gear and a rack, the gear is separately connected to the motor and the rack, and the rack is further connected to the optical display module; and

the IPD adjustment mechanical structure is configured to drive, under driving of the motor, the two optical display modules to move.

19. The head-mounted display device according to claim 18, wherein

there is one motor, and the IPD adjustment mechanical structure is configured to drive, under driving of the motor, the two optical display modules to move simultaneously; or

there are two motors, each motor corresponds to an IPD adjustment mechanical structure, and the two IPD adjustment mechanical structures are connected to the two optical display modules in a one-to-one correspondence; and the IPD adjustment mechanical structure is configured to drive, under driving of the corresponding motor, the corresponding optical display module to move.

20. A computer-readable storage medium, wherein the computer-readable storage medium stores computer instructions, and when the computer instructions are run on a device, the device is enabled to perform the method according to any one of claims 1 to 14.

VR glasses 100

FIG. 1

A　　　　　　　　B

VR glasses 100

Device inter
pupillary distance L

1

2

δX

User inter pupillary
distance D

Left eye　a

b　Right eye

FIG. 2A

A　　Device inter pupillary　　B
distance L

VR glasses 100

1

2

δX

User inter pupillary
distance D

Left eye　a

b　Right eye

FIG. 2B

FIG. 3

341

42

41

Eye

342

342

A

B

FIG. 4A

341

42

43

41

Eye

Move

Move

342

342

342

A

B

C

FIG. 4B

VR glasses 100

FIG. 5A

VR glasses 100

FIG. 5B

VR glasses 100

342
341
34

FIG. 6

Human eye

Ciliary muscle

Cornea

Pupil

Iris

Retina

Crystalline
lens

FIG. 7

FIG. 8

Calibration curve of an IPD distance
and a magnetic induction intensity

Bx

By

Bz

FIG. 9

FIG. 10A

| Current inter pupillary distance value | Adjustment direction | Enter a target inter pupillary distance value to be adjusted to |
|---|---|---|
| X mm | | |

1002

1003    1004

FIG. 10B

| Current inter pupillary distance value | Adjustment direction | Enter a target inter pupillary distance value to be adjusted |
|---|---|---|
| X m | | |

Inter pupillary distance adjustment is completed

1002

1005

FIG. 10C

Inter pupillary distance adjustment is completed, and the current inter pupillary distance value is: X mm

1006

FIG. 10D

Obtain inter pupillary distance adjustment information ~ S1101

Determine a target movement direction of two optical display modules ~ S1102

Control, based on the inter pupillary distance adjustment information and the target movement direction, an IPD adjustment mechanism to operate, to drive the two optical display modules to move ~ S1103

FIG. 11

Control an IPD adjustment mechanism to operate, to drive two optical display modules to move in a direction opposite to a target movement direction beyond a preset distance or to move to an inter pupillary distance limit location ~ S1201

Control, based on inter pupillary distance adjustment information, the IPD adjustment mechanism to operate in an opposite direction, to drive the two optical display modules to move in the target movement direction ~ S1202

FIG. 12

VR glasses 100

Minimum device inter pupillary distance value location

A          B

Direction opposite to a target movement direction

User inter pupillary distance D

a          b

Left eye          Right eye

1          2

FIG. 13A

VR glasses 100

FIG. 13B

VR glasses 100

FIG. 14A

VR glasses 100

A      B   Target
 Device inter pupillary   movement
   distance L     direction

1              2

a User inter pupillary b
   distance D

Left eye        Right eye

FIG. 14B

| Obtain a previous inter pupillary distance adjustment direction | S1501 |

| If the previous inter pupillary distance adjustment direction is the same as a target movement direction, control, based on inter pupillary distance adjustment information, an IPD adjustment mechanism to operate, to drive two optical display modules to move in the target movement direction | S1502 |

| If the previous inter pupillary distance adjustment direction is opposite to a target movement direction, control, based on inter pupillary distance adjustment information and a preset backlash error, an IPD adjustment mechanism to operate, to drive two optical display modules to move in the target movement direction | S1503 |

FIG. 15

Obtain an inter pupillary distance offset of a single eye by using an eye tracking apparatus ⟳ S1601

Obtain a first current detection value of an IPD adjustment sensor, and obtain, according to a calibration table, a first current inter pupillary distance value corresponding to the first current detection value ⟳ S1602

Determine a target inter pupillary distance value and/or a target detection value based on the inter pupillary distance offset of the single eye and the first current inter pupillary distance value ⟳ S1603

Determine a target movement direction of two optical display modules based on at least one of the inter pupillary distance offset of the single eye, the target inter pupillary distance value, or the target detection value ⟳ S1604

Determine a displacement direction and a displacement distance of a motor in an IPD adjustment mechanism based on at least one of the inter pupillary distance offset of the single eye, the target inter pupillary distance value, or the target detection value ⟳ S1605

Control, based on the displacement direction and the displacement distance of the motor, the motor to move, to drive the two optical display modules to move through the motor ⟳ S1606

Obtain a second current detection value of the IPD adjustment sensor, and determine whether a condition for ending inter pupillary distance adjustment is met ⟳ S1607

No

Yes

End

FIG. 16

EP 4 435 492 A1

| Obtain an inter pupillary distance offset of a single eye by using an eye tracking apparatus | S1701 |

| Obtain a first current detection value of an IPD adjustment sensor, and obtain, according to a calibration table, a first current inter pupillary distance value corresponding to the first current detection value | S1702 |

| Determine a target inter pupillary distance value and/or a target detection value based on the inter pupillary distance offset of the single eye and the first current inter pupillary distance value | S1703 |

| Determine a target movement direction of two optical display modules | S1704 |

| Control an IPD adjustment mechanism to operate, to drive the two optical display modules to move in a direction opposite to the target movement direction beyond a preset distance or to move to an inter pupillary distance limit location | S1705 |

| Control, based on at least one of the inter pupillary distance offset of the single eye, the target inter pupillary distance value, or the target detection value, the IPD adjustment mechanism to operate in an opposite direction, to drive the two optical display modules to move in the target movement direction, and determine, in a moving process, whether a condition for ending inter pupillary distance adjustment is met | S1706 |

FIG. 17

Obtain an inter pupillary distance offset of a single eye by using an eye tracking apparatus ⟶ S1801

Obtain a first current detection value of an IPD adjustment sensor, and obtain, according to a calibration table, a first current inter pupillary distance value corresponding to the first current detection value ⟶ S1802

Determine a target inter pupillary distance value and/or a target detection value based on the inter pupillary distance offset of the single eye and the first current inter pupillary distance value ⟶ S1803

Determine a target movement direction of two optical display modules based on at least one of the inter pupillary distance offset of the single eye, the target inter pupillary distance value, or the target detection value ⟶ S1804

Obtain a previous inter pupillary distance adjustment direction, and determine whether the previous inter pupillary distance adjustment direction is the same as the target movement direction ⟶ S1805

No

Yes

Control, based on at least one of the inter pupillary distance offset of the single eye, the target inter pupillary distance value, or the target detection value, an IPD adjustment mechanism to operate, to drive the two optical display modules to move in the target movement direction, and determine, in a moving process, whether a condition for ending inter pupillary distance adjustment is met ⟶ S1806

Control, based on a preset backlash error and at least one of the inter pupillary distance offset of the single eye, the target inter pupillary distance value, or the target detection value, an IPD adjustment mechanism to operate, to drive the two optical display modules to move in the target movement direction, and determine, in a moving process, whether a condition for ending inter pupillary distance adjustment is met ⟶ S1807

FIG. 18

Obtain an inter pupillary distance offset of a first single eye by using an eye tracking apparatus — S1901

Determine a target movement direction of two optical display modules based on the inter pupillary distance offset of the first single eye — S1902

Determine a displacement direction and a displacement distance of a motor in an IPD adjustment mechanism based on the inter pupillary distance offset of the first single eye — S1903

Control, based on the displacement direction and the displacement distance of the motor, the motor to move, to drive the two optical display modules to move through the motor — S1904

Obtain an inter pupillary distance offset of a second single eye by using the eye tracking apparatus, and determine whether a condition for ending inter pupillary distance adjustment is met — S1905

No

Yes

End

FIG. 19

| Obtain a target inter pupillary distance value entered by a user | S2001 |

↓

| Obtain a first current detection value of an IPD adjustment sensor, and obtain, according to a calibration table, a first current inter pupillary distance value corresponding to the first current detection value | S2002 |

↓

| Determine a target movement direction of two optical display modules | S2003 |

↓

| Control an IPD adjustment mechanism to operate, to drive the two optical display modules to move in a direction opposite to the target movement direction beyond a preset distance or to move to an inter pupillary distance limit location | S2004 |

↓

| Control, based on the target inter pupillary distance value and/or a target detection value, the IPD adjustment mechanism to operate in an opposite direction, to drive the two optical display modules to move in the target movement direction, and determine, in a moving process, whether a condition for ending inter pupillary distance adjustment is met | S2005 |

FIG. 20

┌─────────────────────────────────────────┐
│                                          │
│   ┌──────────────────────┐ ⌐ 2101        │
│   │  Processing module   │               │
│   └──────────────────────┘               │
│              │                           │
│   ┌──────────────────────┐ ⌐ 2102        │
│   │  Determining module  │               │
│   └──────────────────────┘               │
│                                          │
│   Inter pupillary distance adjustment apparatus │
└─────────────────────────────────────────┘

FIG. 21

## INTERNATIONAL SEARCH REPORT

| | International application No. |
|---|---|
| | **PCT/CN2023/089307** |

| A. | CLASSIFICATION OF SUBJECT MATTER |
|---|---|

G02B27/01(2006.01)i;  A61B3/11(2006.01)i

According to International Patent Classification (IPC) or to both national classification and IPC

| B. | FIELDS SEARCHED |
|---|---|

Minimum documentation searched (classification system followed by classification symbols)

IPC: G02B27, A06F3, A61B3, H04N13

Documentation searched other than minimum documentation to the extent that such documents are included in the fields searched

Electronic data base consulted during the international search (name of data base and, where practicable, search terms used)

CJFD, CNTXT, ENTXTC: 瞳距, 瞳间距, 瞳孔距离, 自动, 驱动, 马达, 空回, 空程, 回程差, 误差, 方向, 减少, 减小, 降低, 清除, 去除, 距离, 时长, interpupillary distance, lost motion, automatic, drive, motor, lost motion, empty trip, error, deviation, direction, reduce, decrease, minish, eliminate, distance, time

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 106686365 A (GOERTEK TECHNOLOGY CO., LTD.) 17 May 2017 (2017-05-17) description, paragraphs [0053]-[0108], and figures 1-5 | 1-20 |
| Y | CN 109963067 A (ZHEJIANG UNIVIEW TECHNOLOGIES CO., LTD.) 02 July 2019 (2019-07-02) description, paragraphs [0019]-[0043], and figures 1-4 | 1-4, 8-20 |
| Y | CN 112887691 A (GOERTEK OPTICAL TECHNOLOGY CO., LTD.) 01 June 2021 (2021-06-01) description, paragraphs [0056]-[0169], and figures 1-5 | 1, 5-20 |
| A | CN 206906702 U (WEIFANG GOERTEK ELECTRONICS CO., LTD.) 19 January 2018 (2018-01-19) entire document | 1-20 |
| A | CN 106990847 A (PIMAX TECHNOLOGY (SHANGHAI) CO., LTD.) 28 July 2017 (2017-07-28) entire document | 1-20 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **06 July 2023** | **12 July 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/089307**

### C. DOCUMENTS CONSIDERED TO BE RELEVANT

| Category* | Citation of document, with indication, where appropriate, of the relevant passages | Relevant to claim No. |
|---|---|---|
| A | CN 107462992 A (SHENZHEN SKYWORTH NEW WORLD TECHNOLOGY CO., LTD.) 12 December 2017 (2017-12-12)<br>entire document | 1-20 |
| A | CN 107003527 A (SZ DJI TECHNOLOGY CO., LTD.) 01 August 2017 (2017-08-01)<br>entire document | 1-20 |
| A | CN 110646914 A (CHENGDU XGIMI TECHNOLOGY CO., LTD.) 03 January 2020 (2020-01-03)<br>entire document | 1-20 |
| A | CN 111464089 A (CHENGDU XGIMI TECHNOLOGY CO., LTD.) 28 July 2020 (2020-07-28)<br>entire document | 1-20 |
| A | CN 112616020 A (SHENZHEN REOLINK TECHNOLOGY CO., LTD.) 06 April 2021 (2021-04-06)<br>entire document | 1-20 |
| A | US 10386647 B1 (FACEBOOK INC.) 20 August 2019 (2019-08-20)<br>entire document | 1-20 |
| A | US 2017344107 A1 (INTEL CORP.) 30 November 2017 (2017-11-30)<br>entire document | 1-20 |

Form PCT/ISA/210 (second sheet) (July 2022)

## INTERNATIONAL SEARCH REPORT
### Information on patent family members

International application No.

**PCT/CN2023/089307**

| Patent document cited in search report | | | Publication date (day/month/year) | Patent family member(s) | | | Publication date (day/month/year) |
|---|---|---|---|---|---|---|---|
| CN | 106686365 | A | 17 May 2017 | None | | | |
| CN | 109963067 | A | 02 July 2019 | None | | | |
| CN | 112887691 | A | 01 June 2021 | None | | | |
| CN | 206906702 | U | 19 January 2018 | None | | | |
| CN | 106990847 | A | 28 July 2017 | None | | | |
| CN | 107462992 | A | 12 December 2017 | None | | | |
| CN | 107003527 | A | 01 August 2017 | WO | 2018133100 | A1 | 26 July 2018 |
| CN | 110646914 | A | 03 January 2020 | WO | 2021056978 | A1 | 01 April 2021 |
| CN | 111464089 | A | 28 July 2020 | None | | | |
| CN | 112616020 | A | 06 April 2021 | None | | | |
| US | 10386647 | B1 | 20 August 2019 | None | | | |
| US | 2017344107 | A1 | 30 November 2017 | WO | 2017204923 | A1 | 30 November 2017 |

Form PCT/ISA/210 (patent family annex) (July 2022)

**EP 4 435 492 A1**

**Patent documents cited in the description**

- CN 202210451373